# EUROPEAN PATENT SPECIFICATION

(11) **EP 1 558 645 B1**
(45) Date of publication and mention of the grant of the patent: **27.07.2011**
(21) Application number: 03776677.1
(22) Date of filing: 07.11.2003
(51) Int. Cl.: C07K 16/18

(54) **STABILIZED SINGLE DOMAIN ANTIBODIES IN PHARMACEUTICAL COMPOSITION ADAPTED FOR INHALATION**
STABILISIERTE EINZEL-DOMÄNEN-ANTIKÖRPER IN EINER PHARMAZEUTISCHEN ZUSAMMENSETZUNG AUSGESTALTET FÜR INHALATION
ANTICORPS A DOMAINE UNIQUE STABILISES DANS UNE COMPOSITION PHARMACEUTIQUE POUR INHALATION

(30) Priority: 08.11.2002 US 425073 P; 08.11.2002 US 425063 P; 10.01.2003 EP 03447005; 23.06.2003 WO PCT/EP03/06581; 08.07.2003 WO PCT/EP03/07313
(43) Date of publication of application: 03.08.2005
(60) Divisional application: 10183379.6
(73) Proprietor: Ablynx N.V., 9052 Zwijnaarde (BE)
(72) Inventor: SILENCE, Karen, B-3090 Overijse (BE); LAUWEREYS, Marc, B-9450 Haaltert (BE); DREIER, Torsten, 81369 Munich (DE)
(74) Representative: HOFFMANN EITLE
(86) International application number: PCT/BE2003/000193
(87) International publication number: WO 2004/041865

(56) References cited:
- EP-A1- 0 952 218
- WO-A2-99/23221
- ELS CONRATH K ET AL: "Camel single-domain antibodies as modular building units in bispecific and bivalent antibody constructs" JOURNAL OF BIOLOGICAL CHEMISTRY, AMERICAN SOCIETY OF BIOLOGICAL CHEMISTS, BALTIMORE, MD, US, vol. 276, no. 10, 9 March 2001 (2001-03-09), pages 7346-7350, XP002248402 ISSN: 0021-9258
- TANHA J ET AL: "Selection by phage display of llama conventional VH fragments with heavy chain antibody VHH properties" JOURNAL OF IMMUNOLOGICAL METHODS, ELSEVIER, AMSTERDAM, NL, vol. 263, no. 1-2, 1 May 2002 (2002-05-01), pages 97-109, XP004354388 ISSN: 0022-1759

## Description

### FIELD OF THE INVENTION

The present invention provides pharmaceutical compositions as defined in claim 1 which are administered by inhalation.

### BACKGROUND OF THE INVENTION

Polypeptide therapeutics and in particular antibody-based therapeutics have significant potential as drugs because they have exquisite specificity to their target and a low inherent toxicity. However, in order to be effective as therapeutic agent, their pharmacokinetic profile should be optimized. The majority of current antibody applications are for acute disorders. There are however significant opportunities to develop antibody therapeutics for chronic conditions. This will require large doses of protein over a long period of time. Since the cost of antibody production in mammalian cells is high, the development of traditional antibody therapeutics for these applications has been discouraged. An alternative approach has been to express fragments of antibodies such as Fab's or single-chain Fv's in microbial expression systems such as yeast and bacteria. These fragments however have very short circulation times *in vivo.*

Some of the initial approaches to increase the circulation in the bloodstream of proteins and peptides were based on chemical modification, such as pegylation (US 4,179,337). Examples of such products are PEG-Intron, i.e. pegylated interferon alpha-2b for the treatment of HCV, and treatment of chronic disorder with PEG-modified antibodies (A.P Chapman, Adv.Drug Delivery Reviews (2002), 54, 531-545). Such chemical methods, however, suffer from a number of disadvantages, such as inactivation of the target protein or peptide due to the chemical modification of certain amino acid side chains, instability of the target protein/peptide during the chemical reaction.

To overcome these limitations, alternative approaches have been developed, first of all by using non-conventional or modified proteins, secondly by using alternative methods to increase half-life *in vivo.* Stabilisation of the protein drug can therefore be carried out by choosing an inherently stable protein scaffold and providing methods to bind such scaffold to plasma proteins which occur in high concentrations, such as immunoglobulins or albumin. Binding to plasma protein can be an effective means to improving the pharmacokinetic properties of molecules in general. More precisely, binding to albumin to improve the half-life of proteins has been described: M.S. Dennis et al. (J. Biol. Chem. 33, 2383-90, 2002) isolated peptides having affinity for serum albumin. When bound to a Fab molecule, half-lives comparable to pegylated Fab's were obtained. Peptide ligands having affinity for IgG or serum albumin have been disclosed (WO 01/45746). Cemu Bioteknik (Nygren, Wigzell, Uhlen, EP 486525 B1; US 6,267,964) described fusions of active proteins or peptides to polypeptides from bacterial origin that bind to serum albumin (e.g. Staph A). The drawback of these peptide-based approaches is that the peptides have to fold properly and be accessible to binding to serum albumin when fused to the therapeutic protein. Therefore, these peptides are inherently unstable and have affinities in the submicromolar range rather than subnanomolar or low nanomolar range, as is the case with conventional antibodies. As part of a larger protein, such as a conventional antibody molecule, binding of these peptides to albumin may be sterically hindered.

An alternative hybrid molecule with two functional units is based on a heterospecific antibody. Such a hybrid would consist of a bifunctional or heterospecific antibody construct with one entity having specificity and affinity for the target, the second entity having specificity and affinity for a serum protein, such as albumin. However, such heterospecific constructs based on conventional antibodies or Fab fragments have several important drawbacks: these are complex, large molecules composed of two polypeptide chains (VH and VL) and therefore difficult and expensive to produce in high amounts in mammalian expression systems. Furthermore, producing bifunctional antibodies composed of 4 chains (2 VH's and 2 VL's) have the inherent risk of resulting in molecules with the unproductive VH-VL combinations and consequent loss of activity. Several alternatives have been tried with mixed results based on peptide derivatives of conventional antibodies, such as diabodies and bifunctional scFv's (WO0220615; WO9413804; WO9119739; WO9409131) .Holliger et al (Nature Biotech. 15, 632-636, 1979) suggests that binding one of the antibody fragments of a diabody (bispecific construct derived from a conventional antibody) to serum immunoglobulin (IgG) may prolong serum residence time of such diabodies but no suggestion is made that bispecific diabodies may be stabilised using antibodies against a serum protein other than serum IgG. Diabodies are known to be inherently difficult to produce due to stickiness of their exposed surface and due to non-productive associations between the four different V-regions (2 VH+2 VL).

Covalent binding to serum proteins as disclosed in, for example, EP0793506B1, US 5,612,034, 6,103,233, and US20020009441 using reactive groups forming stable covalent bonds to a serum protein or a cell have the inherent disadvantage of unwanted target modification through the reactive groups.

Fusions to large, long lived proteins such as albumin (Syed et al, Blood 89, 3243-3252 (1997), Yeh et al, PNAS 89, 1904-1908 (1992); Celltech (WO0027435)) or N-terminal fusions of albumin polypeptides (Delta Biotech/HGS, US 5,380,712, US 5,766,883) or the Fc portion of IgG (Capon et al, Nature 337, 525-531(1989); Ashkenazi et al, Curr. Op.Immunol. 9, 195-200 (1997)) have been described. Such fusions have the disadvantage of inefficient production and causing unwanted immunological reactions .

A complex of interferon with a monoclonal antibody to increase the serum half-life of interferon has been described in US 5,055,289. Such approach has the inherent risk of impairing the biological activity of the interferon since the size of the construct raises the problem of steric hindrance.

EP 095 221 A describes heterospecific antibodies. Camel-derived single immunoglobulin variable domains have been described in WO 99/23221 and Conrath et al. (J. Biochem. 276 (10), p. 7346).

### THE AIMS OF THE PRESENT INVENTION

It is an aim of the present invention to provide a pharmaceutical composition comprising therapeutic heterospecific antibody polypeptide constructs which overcome the problems of therapeutic antibodies of the art namely, low half-life *in vivo,* poor folding, low expression, and poor stability. It is a further aim of the present invention to provide methods for providing said heterospecific antibodies.

### SUMMARY OF THE INVENTION

### Summary

In one embodiment the present invention relates to a pharmaceutical composition for use in therapy comprising:
- one or more Camelidae VHH or humanized Camelidae VHH each directed against a serum protein of a subject, and
- one or more single domain antibodies each directed against a therapeutic and/or diagnostic target
and a pharmaceutically acceptable vehicle
characterized in that it is administered by inhalation.

In a further embodiment the present invention is a pharmaceutical composition according to the above, wherein the single domain antibodies directed against a target molecule are anti-IgE single domain antibodies.

In a further embodiment the present invention relates to a pharmaceutical composition according to the above, which is formulated in the form a nasal spray (e.g. aerosol), inhaler, pump-type sprayer or aerosol sprayer.

In a further embodiment the present invention relates to a pharmaceutical composition according to the above, which is a lyophilized VHH formulation.

In a further embodiment the present invention relates to a pharmaceutical composition according to the above, comprising a solid carrier.

In a further embodiment the present invention relates to a pharmaceutical composition according to the above, wherein the solid carrier is selected from talc, clay, microcrystalline cellulose, silica and alumina.

In a further embodiment the present invention relates to a pharmaceutical composition according to the above, comprising a liquid carrier.

In a further embodiment the present invention relates to a pharmaceutical composition according to the above, wherein the liquid carrier is selected from water, hydroxyalkyls, glycols and water-alcohol/glycol blends.

In a further embodiment the present invention relates to a pharmaceutical composition according to the above, wherein the single domain antibodies are single domain antibodies derived from a conventional 4-chain antibody (VH) or derived from a heavy chain antibody naturally devoid of light chain (VHH).

In a further embodiment the present invention relates to a pharmaceutical composition according to the above, wherein the single domain antibody is humanized.

In a further embodiment the present invention relates to a pharmaceutical composition according to the above, wherein
- the number of anti-target single domain antibodies is at least two, and
- at least two anti-target single domain antibodies do not share the same sequence, or all anti-target single domain antibodies share the same sequence.

In a further embodiment the present invention relates to a pharmaceutical composition according to the above, wherein the single domain antibodies that are directed against a target have the same sequence or wherein at least two anti-target single domain antibodies have a different sequence but are directed against the same epitopes or different epitopes on the same target.

In a further embodiment the present invention relates to a pharmaceutical composition according to the above, wherein
- the number of anti-serum protein single domain antibodies is at least two, and
- at least two anti-serum protein single domain antibodies do not share the same sequence, or all anti-serum protein single domain antibodies share the same sequence.

In a further embodiment the present invention relates to a pharmaceutical composition according to the above, wherein the single domain antibodies that are directed against a serum protein have the same sequence or wherein at least two anti-serum protein single domain antibodies have a different sequence but are directed against the same epitopes or different epitopes on the same serum protein.

In a further embodiment the present invention relates to a pharmaceutical composition according to the above, wherein the serum protein is selected from serum albumin, serum immunoglobulins, thyroxine-binding protein, transferrin and, fibrinogen or fragment thereof.

In a further embodiment the present invention relates to a pharmaceutical composition according to the above, wherein the anti-serum protein single domain antibody corresponds to a sequence represented by any of SEQ ID NOs: 1 to 4 and 28 to 40; or
a) a homologous sequence of any of SEQ ID NOs: 1 to 4 and 28 to 40, comprising 1, 2, 3, 5, 6, 7, 8, 10 additions, deletions or substitutions of one or more amino acids, which do not substantially alter the functional characteristics of the single domain antibody;
b) a homologous sequence of any of SEQ ID NOs: 1 to 4 and 28 to 40, with a sequence identity of more than 70%, 75%, 80%, 85%, 90%, 95% or 98% with SEQ ID NO: 1 to 4 and 28 to 40;
c) a functional portion of any of SEQ ID NOs: 1 to 4 and 28 to 40 that maintains the interaction with the target with affinity of 10-6 M or better;
d) a functional portion of any of SEQ ID NOs: 1 to 4 and 28 to 40, that comprises a partial deletion of the complete amino acid sequence and still maintains the binding site(s) and protein domain(s) necessary for the binding of and interaction with the target.

In a further embodiment the present invention relates to a pharmaceutical composition according to the above, wherein the heterospecific polypeptide construct is represented by any of SEQ ID NOs: 22 to 24; or
a) a homologous sequence of any of SEQ ID NOs: 22 to 24 comprising 1, 2, 3, 5, 6, 7, 8, 10 additions, deletions or substitutions of one or more amino acids, which do not substantially alter the functional characteristics of the heterospecific polypeptide;
b) a homologous sequence of any of SEQ ID NOs: 22 to 24 with a sequence identity of more than 70%, 75%, 80%, 85%, 90%, 95% or 98% with SEQ ID NO: 22 to 24;
c) a functional portion of any of SEQ ID NOs: 22 to 24 that maintains the interaction with the target with affinity of 10-6 M or better;
d) a functional portion of any of SEQ ID NOs: 22 to 24 that comprises a partial deletion of the complete amino acid sequence and still maintains the binding site(s) and protein domain(s) necessary for the binding of and interaction with the target.

In a further embodiment the present invention relates to a pharmaceutical composition according to the above, for treating, preventing and/or alleviating the symptoms of a disorder relating to allergies.

In a further embodiment the present invention relates to a pharmaceutical composition according to the above, wherein said disorder is selected from hay fever, asthma, atopic dermatitis, allergic skin reaction, allergic eye reactions and food allergies.

### BRIEF DESCRIPTION OF FIGURES AND TABLES

**Figure 1** phage ELISA to show that HSA-specific nanobodies are present in the library as described in Example 4.
**Figure 2** Binding of phages expressing the albumin binders, to plasma blotted on nitrocellulose as described in Example 8.
**Figure 3** Coomassie staining of plasma samples on SDS-PAGE as described in example 8.
**Figure 4** Binding of purified nanobodies to mouse albumin as determined by ELISA as described in Example 10.
**Figure 5** Multiple cloning site of PAX011 for construction of bispecific nanobodies as described in Example 11.
**Figure 6** Sandwich ELISA to show the functionality of both nanobodies in the bispecific construct as described in Example 12.
**Figure 7** Optimization of ELISA to determine nanobody concentration in 10% plasma or in 10% blood as described in Example 14.
**Figure 8** Pharmacokinetics for the monovalent anti-TNF-α nanobody in mice as determined by ELISA as described in Example 16.
**Figure 9** Pharmacokinetics for the bispecific nanobody MSA21/TNF3E in mice as determined by ELISA as described in Example 16.
**Figure 10** Pharmacokinetics for the bispecific nanobody MSA21/TNF3E in mice as determined by ELISA with K208 as compared to URL49 as described in Example 16.
**Figure 11** Pharmacokinetics for the bispecific nanobody MSA24/TNF3E in mice as determined by ELISA as described in Example 16.
**Figure 12** Binding to vWF as determined by ELISA, by purified VHH as described in Example 23.
**Figure 13** ELISA to test inhibition by VHH of binding of vWF to collagen as described in Example 24.
**Figure 14** Sandwich ELISA showing the functionality of both VHHs in a bispecific construct as described in example 27.

**Table 1** Immunization scheme according to Example 1
**Table 2** Results after one and two rounds of panning on mouse serum albumin as described in example 5.
**Table 3** Clones were selected after one and two rounds of selection and periplasmic extracts were prepared. These clones were analyzed in ELISA for binding to human and mouse albumin as described in Example 6.
**Table 4** Sequence listing
**Table 5** Affinities (koff, kon and KD) for albumin binders as determined by BIACORE as described in Example 13.
**Table 6** Results for the LAL-assay for monovalent and bispecific nanobodies after purification on polymyxin as described in Example 15.
**Table 7** Immunization scheme used for llama 002 according to Example 17.
**Table 8** Plaque forming units (pfu) after one or two round(s) of panning on vWF as compared to PBS-casein as described in example 19. Pfu vWF (antigen) divided by pfu casein (a specific binding) = enrichment.
**Table 9** Number of inhibitors versus the number of clones tested after the first and the second round of panning as described in Example 20.
**Table 10** Concentration of VHH (nM) needed to inhibit binding of vWF to collagen by 50% (IC50) as described in Example 23.
**Table 11** IC50 values for bispecific nanobodies against albumin and against vWF as described in Example 28.
**Table 12** Fractional homologies between the amino acid sequences of anti-mouse serum albumin VHHs of the invention.
**Table 13** Fractional homologies between anti-TNF-alpha VHHs of the invention.
**Table 14** Percentage homologies between anti-IFN-gamma VHHs of the invention.
**Table 15** Fractional homologies between anti-vWF VHHs of the invention.

### DETAILED DESCRIPTION OF THE INVENTION

The present invention relates to a pharmaceutical composition comprising a heterospecific polypeptide construct comprising one or more single domain antibodies each directed against a serum protein(s) of a subject, and one or more single domain antibodies each directed against a target molecule(s) as defined in claim 1 and which is administered by by inhalation and the finding that the construct has a significantly prolonged half-life in the circulation of said subject compared with the half-life of the anti-target single domain antibody when not part of such a construct.

Single domain antibodies are antibodies whose complementary determining regions are part of a single domain polypeptide. Examples include, but are not limited to, heavy chain antibodies, antibodies naturally devoid of light chains, single domain antibodies derived from conventional 4-chain antibodies, engineered antibodies and single domain scaffolds other than those derived from antibodies. Single domain antibodies may be any of the art, or any future single domain antibodies. Single domain antibodies may be derived from any species including, but not limited to mouse, human, camel, llama, goat, rabbit, bovine. According to one aspect of the invention, a single domain antibody as used herein is a naturally occurring single domain antibody known as heavy chain antibody devoid of light chains. Such single domain antibodies are disclosed in WO 9404678 for example. For clarity reasons, this variable domain derived from a heavy chain antibody naturally devoid of light chain is known herein as a VHH or nanobody to distinguish it from the conventional VH of four chain immunoglobulins. Such a VHH molecule can be derived from antibodies raised in *Camelidae* species, for example in camel, dromedary, alpaca and guanaco. Other species besides *Camelidae* may produce heavy chain antibodies naturally devoid of light chain; such VHHs are within the scope of the invention.

The one or more single domain antibodies of the polypeptide construct which are directed against a target may be of the same sequence. Alternatively they may not all have the same sequence. It is within the scope of the invention that a heterospecific polypeptide construct comprises anti-target single domain antibodies which do not all share the same sequence, but which are directed against the same target, or fragment thereof, one or more antigens thereof.

Also described are methods for the utilization of a plurality of anti-target and /or anti-serum protein single domain antibodies to increase the avidity and/or affinity of the heterospecific molecule. In this manner, serum half-lives of molecules modified in accordance with the description can be extended. Such modification will modify and/or extend the therapeutic window of a specific therapeutic molecule. This flexibility cannot be achieved with alternative methods in the art, such as when using peptides with specificity to serum proteins, diabodies which are difficult to produce in a multivalent form, chemical modifications (such as pegylation, acylation).

The one or more single domain antibodies of the polypeptide construct which are directed against a serum protein may be of the same sequence. Alternatively they may not all have the same sequence. It is within the scope of the invention that a heterospecific polypeptide construct comprises anti-serum protein single domain antibodies which do not all share the same sequence, but which are directed against serum protein, or fragment thereof, one or more antigens thereof.

In another embodiment, one or more anti-target single domain antibodies of the polypeptide construct may be directed to more than one target (*e.g*. vWF and collagen). Similarly, the anti-serum protein single domain antibodies of the polypeptide construct may be directed against more than one serum protein (*e.g*. serum albumin and fibrinogen).

VHHs, according to the present invention, and as known to the skilled addressee are heavy chain variable domains derived from immunoglobulins naturally devoid of light chains such as those derived from *Camelids* as described in W09404678 (and referred to hereinafter as VHH domains or nanobodies). VHH molecules are about 10x smaller than IgG molecules. They are single polypeptides and very stable, resisting extreme pH and temperature conditions. Moreover, they are resistant to the action of proteases which is not the case for conventional antibodies. Furthermore, *in vitro* expression of VHHs produces high yield, properly folded functional VHHs. In addition, antibodies generated in *Camelids* will recognize epitopes other than those recognised by antibodies generated *in vitro* through the use of antibody libraries or via immunisation of mammals other than *Camelids* (WO 9749805). As such, anti-albumin VHH's may interact in a more efficient way with serum albumin which is known to be a carrier protein, As a carrier protein some of the epitopes of serum albumin may be inaccessible by bound proteins, peptides and small chemical compounds. Since VHH's are known to bind into 'unusual' or non-conventional epitopes such as cavities (WO9749805), the affinity of such VHH's to circulating albumin may be increased.

The present invention also relates to the finding that a heterospecific polypeptide construct comprising one or more VHHs directed against one or more serum proteins of a subject, and one or more VHHs directed against one or more target molecule of said subject surprisingly has significantly prolonged half-life in the circulation of said subject compared with the half-life of the anti-target VHH when not part of said construct. Furthermore, such prolonged half-life is in the range of several days due to the high affinity anti-serum albumin VHH's compared to several hours when using low affinity peptides specific for albumin (Dennis et al, JBC, 277, 35035). The extension of the half-life is demonstrated by the inventors herein, for example, in Example 16, and by the polypeptide represented by SEQ ID NO: 5. Furthermore, the said construct was found to exhibit the same favourable properties of VHHs such as high stability remaining intact in mice for at least 19 days (Example 16), extreme pH resistance, high temperature stability and high target affinity.

A target is any biological substance capable of binding to a heterospecific polypeptide construct of the invention. Targets may be, for example, proteins, peptides, nucleic acids, oligonucleic acids, saccharides, polysaccharides, glycoproteins. Examples include, but are not limited to therapeutic targets, diagnostic targets, receptors, receptor ligands, viral coat proteins, immune system proteins, hormones, enzymes, antigens, cell signaling proteins, or a fragment thereof. Targets may be native protein or a fragment thereof, a homologous sequence thereof, a functional portion thereof, or a functional portion of an homologous sequence.

The properties of single domain antibodies, in particular VHHs, compare favourably with those of antibodies derived from sources such as mouse, sheep, goat, rabbit etc. (*i.e*. traditional antibodies), and humanised derivatives thereof. Traditional antibodies are not stable at room temperature, and have to be refrigerated for preparation and storage, requiring necessary refrigerated laboratory equipment, storage and transport, which contribute towards time and expense. Refrigeration is sometimes not feasible in developing countries. Furthermore, the manufacture or small-scale production of said antibodies is expensive because the mammalian cellular systems necessary for the expression of intact and active antibodies require high levels of support in terms of time and equipment, and yields are very low. Furthermore, traditional antibodies have a binding activity which depends upon pH, and hence are unsuitable for use in environments outside the usual physiological pH range such as, for example, in treating gastric bleeding, gastric surgery. Furthermore, traditional antibodies are unstable at low or high pH and hence are not suitable for oral administration. However, it has been demonstrated that VHHs resist harsh conditions, such as extreme pH, denaturing reagents and high temperatures (Ewert S et al, Biochemistry 2002 Mar 19;41 (11):3628-36), so making them suitable for delivery by oral administration. Furthermore, traditional antibodies have a binding activity which depends upon temperature, and hence are unsuitable for use in assays or kits performed at temperatures outside biologically active-temperature ranges (*e.g*. 37 ± 20°C).

Furthermore VHHs are more soluble, meaning they may be stored and/or administered in higher concentrations compared with conventional antibodies. The polypeptides of the present invention also retain binding activity at a pH and temperature outside those of usual physiological ranges, which means they may be useful in situations of extreme pH and temperature which require a modulation of platelet-mediated aggregation, such as in gastric surgery, control of gastric bleeding, assays performed at room temperature etc. The polypeptides of the present invention also exhibit a prolonged stability at extremes of pH, meaning they would be suitable for delivery by oral administration. The polypeptides of the present invention may be cost-effectively produced through fermentation in convenient recombinant host organisms such as *Escherichia coli* and yeast; unlike conventional antibodies which also require expensive mammalian cell culture facilities, achievable levels of expression are high. Examples of yields of the polypeptides of the present invention are 1 to 10 mg/ml (*E*. *coli*) and up to 1g/l (yeast). The polypeptides of the present invention also exhibit high binding affinity for a broad range of different antigen types, and ability to bind to epitopes not recognised by conventional antibodies; for example they display long CDR-based loop structures with the potential to penetrate into cavities and exhibit enzyme function inhibition. Furthermore, since binding often occurs through the CDR3 loop only, it is envisaged that peptides derived from CDR3 could be used therapeutically (Desmyter et al., J Biol Chem, 2001, 276: 26285-90). The polypeptides of the invention are also able to retain full binding capacity as fusion protein with an enzyme or toxin.

The present invention also relates to a heterospecific polypeptide construct comprising one or more VHHs each directed against one or more serum proteins of a subject, and one or more VHH each directed against one or more target molecules wherein the VHHs belong to the traditional class of *Camelidae* single domain heavy chain antibodies. The present invention also relates to a heterospecific polypeptide construct comprising one or more VHH each directed against one or more serums protein of a subject, and one or more VHH each directed against one or more target molecules wherein the VHHs belong to a class of *Camelidae* single domain heavy chain antibodies that have human-like sequences. A VHH sequence represented by SEQ ID NO: 12 which binds to TNF-alpha and a second VHH which binds to mouse albumin, belongs to this class of VHH peptides. As such, peptides belonging to this class show a high amino acid sequence homology to human VH framework regions and said peptides might be administered to patients directly without expectation of an unwanted immune response therefrom, and without the burden of further humanization.

A human-like class of *Camelidae* single domain antibodies represented by SEQ ID No.1, 3 and 4 have been described in WO03035694 and contain the hydrophobic FR2 residues typically found in conventional antibodies of human origin or from other species, but compensating this loss in hydrophilicity by other substitutions at position 103 that substitutes the conserved tryptophan residue present in VH from double-chain antibodies. As such, peptides belonging to these two classes show a high amino acid sequence homology to human VH framework regions and said peptides might be administered to a human directly without expectation of an unwanted immune response therefrom, and without the burden of further humanisation.

Therefore, one aspect of the present invention allows for the direct administration of an anti-serum albumin polypeptide, wherein the single domain antibodies belong to the humanized class of VHH, and comprise a sequence represented by any of SEQ ID NO: 1, 3 or 4 to a patient in need of the same.

A subject as used herein is any mammal having a circulatory system in which the fluid therein comprises serum proteins. Examples of circulatory system include blood and lymphatic systems. Examples of animals include, but are not limited to, rabbits, humans, goats, mice, rats, cows, calves, camels, llamas, monkeys, donkeys, guinea pigs, chickens, sheep, dogs, cats, horses etc.

One embodiment of the present invention is a heterospecific polypeptide construct comprising at least one single domain antibody directed against a therapeutic target, and at least one single domain antibodies each directed against one or more serum proteins or polypeptides. As already mentioned, the anti-target single domain antibodies may have the same sequence. Alternatively, at least two anti-target single domain antibodies may have the different sequences, but are directed against the same epitope or different epitopes on the same target, fragments thereof, or antigen thereof. Similarly, the anti-serum protein single domain antibodies may have the same sequence. Alternatively, at least two anti-serum protein single domain antibodies may have the different sequences, but are directed against the same epitope or different epitopes on the same serum protein, fragments thereof, or antigen thereof.

In another embodiment of the present invention, where more than one anti-target single domain antibodies is present in the heterospecific polypeptide construct, each anti-target single domain antibody may be directed to a different target (*e.g*. one to vWF and one to collagen). Similarly, where more than one anti-serum protein single domain antibody is present, each anti-serum single domain antibody may be directed to a different serum protein (*e.g*. one to serum albumin and one to fibrinogen).

One embodiment of the invention, is a heterospecific polypeptide, wherein an anti-serum protein single domain antibody corresponds to a sequence represented by any of SEQ ID NOs:1 to 4 and 28 to 40.

The constructs disclosed herein retain the advantageous properties of single domain antibodies (*e.g*. VHHs) and have a prolonged lifetime in the circulation of an individual. Thus, such constructs are able to circulate in the subject's serum for several days, reducing the frequency of treatment, the inconvenience to the subject and resulting in a decreased cost of treatment. Furthermore, it is an aspect of the invention that the half-life of the heterospecific polypeptide constructs may be controlled by the number of anti-serum protein single domain antibodies present in the construct. A controllable half-life is desirable in several circumstances, for example, in the application of a timed dose of a therapeutic heterospecific polypeptide construct, or to obtain a desired therapeutic effect.

According to an aspect of the invention a heterospecific polypeptide construct may be a homologous sequence of a full-length heterospecific polypeptide construct. According to another aspect of the invention, a heterospecific polypeptide construct may be a functional portion of a full-length heterospecific polypeptide construct. According to another aspect of the invention, a heterospecific polypeptide construct may be a homologous sequence of a full-length heterospecific polypeptide construct. According to another aspect of the invention, a heterospecific polypeptide construct may be a functional portion of a homologous sequence of a full-length heterospecific polypeptide construct. According to an aspect of the invention a heterospecific polypeptide construct may comprise a sequence of a heterospecific polypeptide construct.

According to an aspect of the invention a single domain antibody used to form a heterospecific polypeptide construct may be a complete single domain antibody (*e.g*. a VHH) or a homologous sequence thereof. According to another aspect of the invention, a single domain antibody used to form the heterospecific polypeptide construct may be a functional portion of a complete single domain antibody. According to another aspect of the invention, a single domain antibody used to form the heterospecific polypeptide construct may be a homologous sequence of a complete single domain antibody. According to another aspect of the invention, a single domain antibody used to form the heterospecific polypeptide construct may be a functional portion of a homologous sequence of a complete single domain antibody.

A heterospecific polypeptide construct may be an homologous sequence of the parent sequence. According to another aspect of the invention, a heterospecific polypeptide construct may be a functional portion parent sequence. According to another aspect of the invention, a heterospecific polypeptide construct may be a functional portion of a homologous sequence of the parent sequence.

As used herein, an homologous sequence may comprise additions, deletions or substitutions of one or more amino acids, which do not substantially alter the functional characteristics of the polypeptides of the invention. The number of amino acid deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 amino acids.

A homologous sequence of the present invention may include a single domain antibody of the invention which has been humanised.

By humanised is meant mutated so that immunogenicity upon administration in human patients is minor or nonexistent. Humanising a single domain antibody, according to the present invention, comprises a step of replacing one or more of amino acids by their human counterpart as found in the human consensus sequence, without that polypeptide losing its typical character, *i.e*. the humanisation does not significantly affect the antigen binding capacity of the resulting polypeptide. Such methods are known by the skilled addressee. A humanisation technique applied to *Camelidae* VHHs may also be performed by a method comprising the replacement of any of the following residues either alone or in combination: some VHH contain typical *Camelidae* hallmark residues at position 37, 44, 45 and 47 with hydrophilic characteristics. Replacement of the hydrophilic residues by human hydrophobic residues at positions 44 and 45 (E44G and R45L) did not have an effect on binding and/or inhibition. Further humanization may be required by substitution of residues in FR 1 , such as position 1, 5, 28 and 30; FR3, such as positions 74, 75, 76, 83, 84, 93 and 94; and FR4, such as position 103, 104, 108 and 111 (all numbering according to the Kabat).

One embodiment is a method for humanizing a VHH comprising the steps of replacing of any of the following residues either alone or in combination:
FR1 position 1, 5, 28 and 30,
the hallmark amino acid at position 44 and 45 in FR2,
FR3 residues 74, 75, 76, 83, 84, 93 and 94,
and positions 103, 104, 108 and 111 in FR4 ;
(numbering according to the Kabat numbering).

Some *Camelidae* VHH sequences display a high sequence homology to human VH framework regions and therefore said VHH might be administered to patients directly without expectation of an immune response therefrom, and without the additional burden of humanisation. Therefore, one aspect of the present invention allows for the formation of a heterospecific polypeptide construct without humanisation of the VHH, when said VHH exhibit high homology to human VH framework regions.

A homologous sequence of the present invention may be a sequence of the invention derived from another species such as, for example, camel, llama, dromedary, alpaca, guanaco etc.

Where homologous sequence indicates sequence identity, it means a sequence which presents a high sequence identity (more than 70%, 75%, 80%, 85%, 90%, 95% or 98% sequence identity) with a single domain antibody of the invention, and is preferably characterised by similar properties of the parent sequence, namely affinity, said identity calculated using known methods.

A homologous sequence may refer to nucleotide sequences of more than 50, 100, 200, 300, 400, 500, 600, 800 or 1000 nucleotides able to hybridise to the reverse-complement of the nucleotide sequence capable of encoding a native sequence under stringent hybridisation conditions (such as the ones described by SAMBROOK et al., Molecular Cloning, Laboratory Manuel, Cold Spring, Harbor Laboratory press, New York).

As used herein, a functional portion refers to a single domain antibody of sufficient length such that the interaction of interest is maintained with affinity of 1 x 10⁻⁶ M or better.

Alternatively a functional portion of a single domain antibody of the invention comprises a partial deletion of the complete amino acid sequence and still maintains the binding site(s) and protein domain(s) necessary for the binding of and interaction with the target or serum protein.

As used herein, a functional portion of a single domain antibody of the invention refers to less than 100% of the sequence (*e.g*., 99%, 90%, 80%, 70%, 60%, 50%, 40%, 30%, 20%, 10%, etc.), but comprising 5 or more amino acids or 15 or more nucleotides.

A portion of a single domain antibody of the invention refers to less than 100% of the sequence (*e.g*., 99%, 90%, 80%, 70%, 60%, 50%, 40%, 30%, 20%, 10%, etc.), but comprising 5 or more amino acids or 15 or more nucleotides.

Targets as mentioned herein such as TNF-alpha, IFN-gamma receptor, serum proteins (*e.g*. serum albumin, serum immunoglobulins, thyroxine-binding protein, transferrin, fibrinogen) and IFN-gamma may be fragments of said targets. Thus a target is also a fragment of said target, capable of eliciting an immune response. A target is also a fragment of said target, capable of binding to a single domain antibody raised against the full length target.

A fragment as used herein refers to less than 100% of the sequence (e.g., 99%, 90%, 80%, 70%, 60%, 50%, 40%, 30%, 20%, 10% etc.), but comprising 5, 6, 7, 8, 9, 10, 12, 13, 14, 15, 16, 17, 18, 19, 20, 21, 22, 23, 24, 25 or more amino acids. A fragment is of sufficient length such that the interaction of interest is maintained with affinity of 1 x 10⁻⁶ M or better.

A fragment as used herein also refers to optional insertions, deletions and substitutions of one or more amino acids which do not substantially alter the ability of the target to bind to a single domain antibody raised against the wild-type target. The number of amino acid insertions deletions or substitutions is preferably up to 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69 or 70 amino acids.

The serum protein may be any suitable protein found in the serum of subject, or fragment thereof. In one aspect of the invention, the serum protein is serum albumin, serum immunoglobulins, thyroxine-binding protein, transferrin, or fibrinogen. Depending on the intended use such as the required half-life for effective treatment and/or compartimentalisation of the target antigen, the VHH-partner can be directed to one of the above serum proteins.

A single domain antibody directed against a target means single domain antibody that it is capable of binding to its target with an affinity of better than 10⁻⁶ M.

The heterospecific polypeptide constructs disclosed herein may be made by the skilled artisan according to methods known in the art or any future method. For example, VHHs may be obtained using methods known in the art such as by immunising a camel and obtaining hybridomas therefrom, or by cloning a library of single domain antibodies using molecular biology techniques known in the art and subsequent selection by using phage display.

The anti-serum protein single domain antibody may be directed against a polypeptide of a serum protein or a whole protein. The anti-target single domain antibody may be directed against a polypeptide of said target of the whole target. Methods for scanning a protein for immunogenic polypeptides are well known in the art.

The single domain antibodies may be joined using methods known in the art or any future method. For example, they may be fused by chemical cross-linking by reacting amino acid residues with an organic derivatising agent such as described by Blattler et al, Biochemistry 24,1517-1524; EP294703. Alternatively, the single domain antibody may be fused genetically at the DNA level *i.e*. a polynucleotide construct formed which encodes the complete polypeptide construct comprising one or more anti-target single domain antibodies and one or more anti-serum protein single domain antibodies. A method for producing bivalent or multivalent VHH polypeptide constructs is disclosed in PCT patent application WO 96/34103. One way of joining multiple single domain antibodies is via the genetic route by linking single domain antibody coding sequences either directly or *via* a peptide linker. For example, the C-terminal end of the first single domain antibody may be linked to the N-terminal end of the next single domain antibody. This linking mode can be extended in order to link additional single domain antibodies for the construction and production of tri-, tetra-, etc. functional constructs.

The present invention is the administration of heterospecific polypeptide constructs according to the invention by inhalation which avoids the need for injection. Conventional antibody-based therapeutics have significant potential as drugs because they have exquisite specificity to their target and a low inherent toxicity, however, they have one important drawback: these are complex, large molecules and therefore relatively unstable, and they are sensitive to breakdown by proteases. This means that conventional antibody drugs cannot be administered by inhalation because they are not resistant to the low pH at these sites, the action of proteases at these sites and in the blood and/or because of their large size. They have to be administered by injection (intravenously, subcutaneously, etc.) to overcome some of these problems. Administration by injection requires specialist training in order to use a hypodermic syringe or needle correctly and safely. It further requires sterile equipment, a liquid formulation of the therapeutic polypeptide, vial packing of said polypeptide in a sterile and stable form and, of the subject, a suitable site for entry of the needle. Furthermore, subjects commonly experience physical and psychological stress prior to and upon receiving an injection. The present invention overcomes these problems of the prior art, by providing the heterospecific polypeptides constructs in the pharmaceutical composition of the present invention. Said constructs are sufficiently small, resistant and stable to be delivered by inhalation substantial without loss of activity. The heterospecific polypeptides constructs of the present invention avoid the need for injections, are not only cost/time savings, but are also more convenient and more comfortable for the subject.

The present invention is a pharmaceutical composition comprising heterospecific polypeptide construct comprising at least one single domain antibody directed against a target comprising at least one single domain antibody directed against a target, for use in treating, preventing and/or alleviating the symptoms of disorders susceptible to modulation by an anti-target therapeutic compound delivered to the nose, upper respiratory tract and/or lung by inhalation.

In a non-limiting example, a formulation according to the invention, comprises a heterospecific polypeptide construct as disclosed herein directed against one or more targets in the form of a nasal spray (e.g. an aerosol) or inhaler. For administration by inhalation. Since the construct is small, it can reach its target much more effectively than therapeutic IgG molecules.

An aspect of the invention is a method for treating, preventing and/or alleviating the symptoms of disorders susceptible to modulation by a therapeutic compound delivered to the upper respiratory tract and lung, by administering to a subject a heterospecific polypeptide construct as disclosed herein wherein one or more single domain antibodies are specific for an antigen related to the disorder, by inhalation through the mouth or nose.

Another aspect of the invention is a dispersible VHH composition, in particular dry powder dispersible VHH compositions, such as those described in US 6514496. These dry powder compositions comprise a plurality of discrete dry particles with an average particle size in the range of 0.4-10 mm. Such powders are capable of being readily dispersed in an inhalation device. VHH's are particularly suited for such composition as lyophilized material can be readily dissolved (in the lung subsequent to being inhaled) due to its high solubilisation capacity (Muyldermans, S., Reviews in Molecular Biotechnology, 74, 277-303, (2001)).

Another embodiment of the present invention is a use of a heterospecific polypeptide construct as disclosed herein for the preparation of a medicament for treating, preventing and/or alleviating the symptoms of disorders susceptible to modulation by an anti-target therapeutic compound delivered to the nose, upper respiratory tract and/or lung by inhalation without being inactivated.

An aspect of the invention is a method for delivering an anti-target therapeutic compound to the nose, upper respiratory tract and lung, by administering to the nose, upper respiratory tract and/or lung of a subject a heterospecific polypeptide construct comprising one or more single domain antibodies directed against said target by inhalation.

An aspect of the invention is a method for delivering an anti-target therapeutic compound to the nose, upper respiratory tract and/or lung by inhalation without being inactivated, by administering to the nose, upper respiratory tract and/or lung of a subject a heterospecific polypeptide construct comprising one or more single domain antibodies directed against said target

An aspect of the invention is a method for delivering an anti-target therapeutic compound to the bloodstream of a subject without being inactivated by administering to the nose, upper respiratory tract and/or lung of a subject by inhalation a heterospecific polypeptide construct comprising one or more single domain antibodies directed against said target.

In another embodiment of the present invention, a heterospecific polypeptide construct further comprises a carrier single domain antibody (e.g. VHH) which acts as an active transport carrier for transport said heterospecific polypeptide construct, the lung lumen to the blood.

A polypeptide construct further comprising a carrier binds specifically to a receptor present on the mucosal surface (bronchial epithelial cells) resulting in the active transport of the polypeptide from the lung lumen to the blood. The carrier single domain antibody may be fused to the polypeptide construct. Such fusion constructs made using methods known in the art and are describe herein. The "carrier" single domain antibody binds specifically to a receptor on the mucosal surface which induces an active transfer through the surface.

Another aspect is a method to determine which single domain antibodies (e.g. VHHs) are actively transported into the bloodstream upon nasal administration. Similarly, a naive or immune VHH phage library can be administered nasally, and after different time points after administration, blood or organs can be isolated to rescue phages that have been actively transported to the bloodstream. A non-limiting example of a receptor for active transport from the lung lumen to the bloodstream is the Fc receptor N (FcRn). One aspect of the invention includes the VHH molecules identified by the method. Such VHH can then be used as a carrier VHH for the delivery of a therapeutic VHH to the corresponding target in the bloodstream upon nasal administration.

Another embodiment of the present invention is a heterospecific polypeptide construct as disclosed herein for use in treating, preventing and/or alleviating the symptoms of a disorder requiring a therapeutic or diagnostic compound which is not rapidly cleared from the circulation. An aspect of the invention is the use of a said construct for the preparation of a medicament for treating, preventing and/or alleviating the symptoms of a disorder requiring a therapeutic or diagnostic compound which is not rapidly cleared from the circulation. Another aspect of the invention is a method for treating, preventing and/or alleviating the symptoms of a disorder requiring a therapeutic or diagnostic compound which is not rapidly cleared from the circulation by administering a heterospecific polypeptide construct as disclosed herein to an individual. According to the present invention, the anti-target single domain antibody of said heterospecific polypeptide is directed against a target involved in a cause or a manifestation of said disorder, or involved in causing symptoms thereof. By using a heterospecific polypeptide construct of the present invention to treat or diagnose an aforementioned disorder, the depletion of said construct is retarded.

Another embodiment of the present invention is a heterospecific polypeptide construct as disclosed herein for use in treating, preventing and/or alleviating the symptoms of a disorder requiring a therapeutic or diagnostic compound which remains active in the circulation for extended periods of time. An aspect of the invention is the use of said construct for the preparation of a medicament for treating, preventing and/or alleviating the symptoms of a disorder requiring a therapeutic or diagnostic compound which remains active in the circulation for extended periods of time. Another aspect of the invention is a method for treating, preventing and/or alleviating the symptoms of a disorder requiring a therapeutic or diagnostic compound that is able to circulate in the patients serum for several days, by administering a heterospecific polypeptide construct as disclosed herein to an individual. According to the present invention, the anti-target single domain antibody of said heterospecific polypeptide is directed against a target involved in a cause or a manifestation of said disorder, or involved in causing symptoms thereof. By using a heterospecific polypeptide construct of the present invention to treat or diagnose an aforementioned disorder, the frequency of treatment is reduced, so resulting in a decreased cost of treatment.

Another embodiment of the present invention is a heterospecific polypeptide construct as disclosed herein for use in treating, preventing and/or alleviating the symptoms of a disorder relating to allergies. An aspect of the invention is the use of said construct for the preparation of a medicament for treating, preventing and/or alleviating the symptoms of a disorder relating to allergies. Another aspect of the invention is a method for treating, preventing and/or alleviating the symptoms of a disorder relating to allergies, by administering a heterospecific polypeptide construct as disclosed herein to an individual. According to the present invention, the anti-target single domain antibody of said heterospecific polypeptide is directed against a target involved in a cause or a manifestation of said disorder, or involved in causing symptoms thereof.

The above aspects and embodiments of the invention also apply when an anti-serum single domain antibody of the aforementioned heterospecific polypeptide constructs corresponds to a sequence represented by SEQ ID NOs: 1 to 4, a homologous sequence thereof, a functional portion thereof, or a homologous sequence of a functional portion.

The above aspects and embodiments of the invention also apply when a heterospecific polypeptide construct of the invention corresponds to a sequence represented by any of SEQ ID NOs: 5 to 18, a homologous sequence thereof, a functional portion thereof, or a homologous sequence of a functional portion. Said sequences comprise an anti-TNF-alpha *Camelidae* VHH.

The above aspects and embodiments of the invention also apply when an heterospecific polypeptide constructs of the invention corresponds to a sequence represented by any of SEQ ID NOs: 19 to 21 a homologous sequence thereof, a functional portion thereof, or a homologous sequence of a functional portion. Said sequences comprise an anti-vWF *Camelidae* VHH.

The above aspects and embodiments of the invention also apply when an heterospecific polypeptide constructs of the invention corresponds to a sequence represented by any of SEQ ID NOs: 22 to 24 a homologous sequence thereof, a functional portion thereof. Said sequences comprise an anti-IgE *Camelidae* VHH.

The above aspects and embodiments of the invention also apply when an heterospecific polypeptide construct according to the invention corresponds to a sequence represented by any of SEQ ID NOs:25 to 27, a homologous sequence thereof, a functional portion thereof, or a homologous sequence of a functional portion. Said sequences comprise an anti-Interferon-gamma *Camelidae* VHH.

A non-limiting example, in relation to allergies, of a target against which an anti-target single domain antibody may be directed is IgE. During their lifetime, subjects can develop an allergic response to harmless parasites such as *Dermatophagoides pteronyssinus,* the house dust mite or to substances such as clumps, plastics, metals. This results in an induction of IgE molecules that initiates a cascade of immunological responses. One aspect of the present invention is a heterospecific polypeptide construct comprising one or more anti-IgE single domain antibodies fused to one or more anti-serum protein single domain antibodies. In one aspect of the invention, said anti-IgE single domain antibodies prevents the interaction of IgE with their receptor(s) on mast cells and basophils, so blocking initiation of the immunological cascade and a subsequent allergic reaction. In another aspect an anti-serum protein single domain antibody is directed to one of the subject's serum proteins. A heterospecific polypeptide construct as disclosed herein thus reduces or prevents an allergic response due to common or unusual allergens. Furthermore, the construct has a prolonged lifetime in the blood so increasing the therapeutic window.

Tumor necrosis factor alpha (TNF-alpha) is believed to play an important role in various diseases, for example in inflammatory diseases such as rheumatoid arthritis, Crohn's disease, ulcerative colitis and multiple sclerosis. Both TNF-alpha and the receptors (CD120a, CD120b) have been studied in great detail. TNF-alpha in its bioactive form is a trimer and the groove formed by neighboring subunits is important for the cytokine-receptor interaction. Several strategies to antagonize the action of the cytokine have been developed and are currently used to treat various disease states.

A TNF inhibitor which has sufficient specificity and selectivity to TNF may be an efficient prophylactic or therapeutic pharmaceutical compound for preventing or treating inflammatory diseases. However, it is extremely difficult and a lengthy process to develop a small chemical entitiy (NCE) with sufficient potency and selectivity to such target sequence. Antibody-based therapeutics on the other hand have significant potential as drugs because they have exquisite specificity to their target and a low inherent toxicity. In addition, the development time can be reduced considerably when compared to the development of new chemical entities (NCE's). However, conventional antibodies are difficult to elicit against multimeric proteins where the receptor-binding domain of the ligand is embedded in a groove, as is the case with TNF-alpha.

The heterospecific polypeptide constructs of the present invention, wherein the anti-target single domain antibody is directed against TNF-alpha overcome the problems experienced using peptide therapeutics of the art because of the properties such as stability, size, and reliable expressioin. Furthermore, the inventiors have found that, despite presence of a groove in multimeric TNF-alpha, the heterospecific polypeptide constructs are still able to achieve strong binding to TNF-alpha

Another embodiment is a heterospecific polypeptide construct as disclosed herein for use in treating, preventing and/or alleviating the symptoms of a disorder mediated by inflammatory molecules. An aspect of the invention is the use of said construct for the preparation of a medicament for treating, preventing and/or alleviating the symptoms of a disorder mediated by inflammatory molecules. Another aspect of the invention is a method for treating, preventing and/or alleviating the symptoms of a disorder mediated by inflammatory molecules, by administering a heterospecific polypeptide construct as disclosed herein to an individual. According to the present invention, an anti-target single domain antibody of said heterospecific polypeptide is directed against a target involved in a cause or a manifestation of said disorder, or involved in causing symptoms thereof.

A target against which a single domain antibody of a heterospecific polypeptide construct is directed is tumor necrosis factor alpha (TNF-alpha). TNF-alpha is believed to play an important role in various disorders, for example in inflammatory disorders such as rheumatoid arthritis, Crohn's disease, ulcerative colitis and multiple sclerosis.

Anti-target single domain antibodies may be directed against whole TNF-alpha or a fragment thereof, or a fragment of a homologous sequence thereof.

A heterospecific polypeptide construct comprising one or more anti-TNF-alpha single domain antibody fused to one or more anti-serum protein single domain antibody, the sequences of said heterospecific polypeptide corresponding to any of SEQ ID NOs: 5 to 18. The anti-TNF-alpha single domain antibodies therein are derived from *Camelidae* heavy chain antibodies (VHHs), which bind to TNF-alpha.

A heterospecific polypeptide construct comprising one or more anti-TNF-alpha single domain antibodies fused to one or more anti-serum protein single domain antibodies for use in treating, preventing and/or alleviating the symptoms of inflammatory disorders. TNF-alpha is involved in inflammatory processes, and the blocking of TNF-alpha action can have an anti-inflammatory effect, which is highly desirable in certain disorder states such as, for example, Crohn's disease. Oral delivery of these heterospecific polypeptide construct results in the delivery of such molecules in an active form in the colon at sites that are affected by the disorder. These sites are highly inflamed and contain TNF-alpha producing cells. These heterospecific polypeptide constructs can neutralise the TNF-alpha locally, avoiding distribution throughout the whole body and thus limiting negative side-effects. Genetically modified microorganisms such as *Micrococcus lactis* are able to secrete antibody fragments. Such modified microorganisms can be used as vehicles for local production and delivery of antibody fragments in the intestine. By using a strain which produces a TNF-alpha-neutralising heterospecific polypeptide construct, inflammatory bowel disorder could be treated.

Another aspect is a heterospecific polypeptide construct comprising one or more anti-TNF-alpha single domain antibodies fused to one or more anti-serum protein single domain antibodies for use in the treatment, prevention and/or alleviation of disorders relating to inflammatory processes, wherein said heterospecific polypeptide construct is administered by inhalation.

Another aspect is the use of a heterospecific polypeptide construct comprising one or more anti-TNF-alpha single domain antibodies fused to one or more anti-serum protein single domain antibodies for the preparation of a medicament for the treatment, prevention and/or alleviation of disorders relating to inflammatory processes, wherein said heterospecific polypeptide construct is administered by inhalation.

Another aspect of the invention is a method of treating, preventing and/or alleviating disorders relating to inflammatory processes, comprising administering to a subject a heterospecific polypeptide construct comprising one or more anti-TNF-alpha single domain antibodies fused to one or more anti-serum protein single domain antibodies by inhalation.

Another aspect of the invention is a heterospecific polypeptide construct comprising one or more anti-TNF-alpha single domain antibodies fused to one or more anti-serum protein single domain antibodies for use in the treatment, prevention and/or alleviation of disorders relating to inflammatory processes.

Another aspect of the invention is a heterospecific polypeptide construct comprising one or more anti-TNF-alpha single domain antibodies fused to one or more anti-serum protein single domain antibodies for the preparation of a medicament for the treatment, prevention and/or alleviation of disorders relating to inflammatory processes.

It is an aspect of the invention that the anti-TNF-alpha single domain antibodies of the present invention may be derived from VHHs of any class. For example, they may be derived from a class of VHHs with high homology to the human VH sequence, or may be derived from any of the other classes of VHHs, including the major class of VHH. These VHHs include the full length *Camelidae* VHHs, domains and may comprise a human Fc domain if effector functions are needed.

The above aspects and embodiments apply to a heterospecific polypeptide construct comprising one or more anti-TNF-alpha single domain antibodies fused to one or more anti-serum protein single domain antibodies, wherein said heterospecific polypeptide corresponds to a sequence represented by any of SEQ ID NOs: 5 to 18, a homologous sequence thereof, a functional portion thereof, of a homologous sequence of a functional portion thereof. SEQ ID NOs: 5 to 18 comprise anti-TNF alpha *Camelidae* VHH and anti-mouse serum albumin *Camelidae* VHH.

The above aspects and embodiments apply to a heterospecific polypeptide construct comprising one or more anti-TNF-alpha single domain antibodies fused to one or more anti-serum protein single domain antibodies wherein said anti-serum protein single domain antibodies correspond to any of SEQ ID NOs: 1 to 4 (anti-serum protein *Camelidae* VHHs), a homologous sequence thereof, a functional portion thereof, of a homologous sequence of a functional portion thereof.

The inventors have found that a heterospecific polypeptide construct comprising a sequence corresponding to any of SEQ ID NOs: 5 to 18 surprisingly exhibits higher than expected affinity towards its target and prolonged half-life in the circulatory system.

Platelet-mediated aggregation is the process wherein von Willebrand Factor (vWF)-bound collagen adheres to platelets and/or platelet receptors (examples of both are gpIa/IIa, gplb, or collagen), ultimately resulting in platelet activation. Platelet activation leads to fibrinogen binding, and finally to platelet aggregation. The ability to disrupt platelet-mediated aggregation has many applications including the treatment of disease as mentioned below. Since the heterospecific polypeptide constructs of the invention effective prevent clotting, and the half-life thereof is controllable, they may be used for surgical procedures, for example, which require an inhibition of platelet-mediated aggregation for a limited time period.

Monovalent single domain antibodies such as VHHs show surprisingly high platelet aggregation inhibition in experiments to measure platelet aggregation inhibition under high shear: 50% inhibition of platelet aggregation was obtained at a concentration between 4 and 25 nM. In comparison, the Fab fragment derived from a vWF-specific antibody inhibiting the interaction with collagen, 82D6A3, inhibits 50% of platelet aggregation at approximately a twenty-fold higher concentration (Vanhoorelbeke K. et al, Journal of Biological Chemistry, 2003, 278: 37815-37821). These results were unexpected given that the IC50 values for the monovalent VHH's are up to 225 times fold worse in ELISA then the IC50 value of the IgG of 82D6A3.

This clearly shows that IgG antibodies is not suited to interaction with macromolecules which are starting, or are in the process of aggregating, such as those involved in platelet-mediated aggregation. vWF makes multimers of up to 60 monomers (final multimers of up to 20 million dalton in size). Indeed, it has been shown that not all A3 domains are accessible to 82D6A3 (Dongmei WU, Blood, 2002, 99, 3623 to 3628). Furthermore the large size of conventional antibodies, would restrict tissue penetration, for example, during platelet-mediated aggregation at the site of a damaged vessel wall.

The structure of single domain antibodies, in particular is unique. For example VHH molecules derived from *Camelidae* antibodies are among the smallest intact antigen-binding domains known (approximately 15 kDa, or 10 times smaller than a conventional IgG) and hence are well suited towards delivery to dense tissues and for accessing the limited space between macromolecules participating in or starting the process of platelet mediated aggregation.

To our knowledge, this is the first time that experiments show, that the small size of a VHH is advantageous over a large intact antibody for inhibition of interactions between such large macromolecules.

Despite the small size of nanobodies, and thus advantages for penetration, it is still surprising that such a small molecule can inhibit interactions between large polymers such as vWF (up to 60 monomers) and collagen and with such a high efficiency. It has been described that only the large multimeric forms of vWF are hemostatically active (Furlan, M,. 1996, Ann. Hematol. 72:341-348). Binding of multimeric vWF to collagen occurs with ~100-fold higher affinitythan binding of monomeric vWF fragments.

The results from the high shear experiments indicate that a lower dose will be needed for administration to patients. Therefore, fewer side effects are expected (such as immunogenicity or bleeding problems).

It is an aspect to provide heterospecific polypeptide constructs which modulate processes which comprise platelet-mediated aggregation such as, for example, vWF-collagen binding, vWF-platelet receptor adhesion, collagen-platelet receptor adhesion, platelet activation, fibrinogen binding and/or platelet aggregation. Said heterospecific polypeptide constructs are derived from single domain antibodies directed towards vWF, vWF A1 or A3 domains, gplb or collagen.

Anti-target single domain antibodies may be directed against whole vWF, vWF A1 or A3 domains, gplb or collagen or a fragment thereof, or a fragment of a homologous sequence thereof.

A target against which a heterospecific polypeptide construct comprising one or more anti-target single domain antibodies fused to one or more anti-serum protein single domain antibodies is directed is von Willebrand factor (vWF). According to another aspect of the invention, the target is vWF A1 or A3 domains. According to another aspect of the invention, the target is gplb. According to another aspect of the invention, the target is gpla/IIA. According to another aspect of the invention, the target is collagen.

One aspect relates to a heterospecific polypeptide construct comprising one or more anti-vWF single domain antibodies fused to one or more anti-serum protein VHHs, the sequences of said heterospecific polypeptide corresponding to any of SEQ ID NOs: 19 to 21. The anti-vWF single domain antibodies therein are derived from *Camelidae* heavy chain antibodies (VHHs), which bind to vWF.

One embodiment is a heterospecific polypeptide construct comprising one or more anti-target single domain antibodies fused to one or more anti-serum protein single domain antibodies target, wherein the target is any of vWF, vWF A1 or A3 domains, gplb or collagen for use in treating, preventing and/or alleviating the symptoms of disorders or conditions relating to platelet-mediated aggregation or dysfunction thereof. Said disorders include transient cerebral ischemic attack, unstable angina pectoris, cerebral infarction, myocardial infarction, peripheral arterial occlusive disease, restenosis. Said conditions include those arising from coronary by-pass graft, coronary artery valve replacement and coronary interventions such angioplasty, stenting, or atherectomy.

One aspect is a heterospecific polypeptide construct comprising one or more anti-target single domain antibodies fused to one or more anti-serum protein single domain antibodies, wherein the target is any of vWF, vWF A1 or A3 domains or collagen for use in the treatment, prevention and/or alleviation of disorders or conditions relating to platelet-mediated aggregation or dysfunction thereof, wherein said heterospecific polypeptide construct is administered by inhalation.

Another aspect of the invention is the use of a heterospecific polypeptide construct comprising one or more anti-target single domain antibodies fused to one or more anti-serum protein single domain antibodies target, wherein the target is any of vWF, vWF A1 or A3 domains or collagen for the preparation of a medicament for the treatment, prevention and/or alleviation of disorders or conditions relating to platelet-mediated aggregation or dysfunction thereof, wherein said heterospecific polypeptide construct is administered by inhalation.

Another aspect of the invention is a method of treating, preventing and/or alleviating disorders or conditions relating to relating to platelet-mediated aggregation or dysfunction thereof, comprising administering to a subject a heterospecific polypeptide construct comprising one or more anti-target single domain antibodies fused to one or more anti-serum protein single domain antibodies target, wherein the target is any of vWF, vWF A1 or A3 domains or collagen, wherein said heterospecific polypeptide construct is administered by inhalation.

Another aspect of the invention is a heterospecific polypeptide construct comprising one or more anti-target single domain antibodies fused to one or more anti-serum protein single domain antibodies, wherein the target is any of vWF, vWF A1 or A3 domains or collagen for use in the treatment, prevention and/or alleviation of disorders or conditions relating to platelet-mediated aggregation or dysfunction thereof.

Another aspect of the invention is a use of a heterospecific polypeptide construct comprising one or more anti-target single domain antibodies fused to one or more anti-serum protein single domain antibodies, wherein the target is any of vWF, vWF A1 or A3 domains or collagen for the preparation of a medicament for the treatment, prevention and/or alleviation of disorders or conditions relating to platelet-mediated aggregation or dysfunction thereof.

It is an aspect that the anti-vWF, anti-vWF A1 or anti-vWF A3 or anti-collagen VHHs of the present invention may be derived from VHHs of any class. For example, they may be derived from the class of VHHs with high homology to the human VH sequence, or may be derived from any of the other classes of VHHs, including the major class of VHH. These VHHs include the full length *Camelidae* VHHs, domains and may comprise a human Fc domain if effector functions are needed.

The above aspects and embodiments apply to a heterospecific polypeptide construct comprising one or more anti-vWF single domain antibodies wherein said heterospecific polypeptide corresponds to a sequence represented by any of SEQ ID NOs: 19 to 21, a homologous sequence thereof, a functional portion thereof, of a homologous sequence of a functional portion thereof. SEQ ID NOs: 19 to 21 comprise anti-vWF VHH and anti-mouse serum albumin VHH.

The above aspects and embodiments apply to a heterospecific polypeptide construct comprising one or more anti-target single domain antibodies fused to one or more anti-serum protein single domain antibodies, wherein the target is any of vWF, vWF A1 or A3 domains, gplb or collagen and wherein said anti-serum protein single domain antibodies correspond to any of SEQ ID NOs: 1 to 4, a homologous sequence thereof, a functional portion thereof, of a homologous sequence of a functional portion thereof.

During their lifetime, subjects may develop an allergic response to harmless parasites (e.g. *Dermatophagoides pteronyssinus,* house dust mite) or substances (clumps, plastics, metals). This results in the induction of IgE molecules that initiate a cascade of immunological responses. One aspect of the present invention is a heterospecific polypeptide construct comprising one or more anti-IgE single domain antibodies, said heterospecific polypeptide construct preventing the interaction of IgEs with their receptor(s) on mast cells and basophils. As such they prevent the initiation of the immunological cascade, an allergic reaction.

According to one aspect of the invention, a target against which a heterospecific polypeptide construct comprising one or more anti-target single domain antibodies fused to one or more anti-serum protein single domain antibodies is directed is IgE. Said antibodies may be directed against whole IgE or a fragment thereof, or a fragment of a homologous sequence thereof.

One aspect of the present invention relates to a heterospecific polypeptide construct comprising one or more anti-IgE single domain antibodies fused to one or more anti-serum protein single domain antibodies, wherein the sequences of said heterospecific polypeptide corresponding to any of SEQ ID NOs: 22 to 24. The anti-IgE single domain antibodies therein are derived from *Camelidae* heavy chain antibodies (VHHs), which bind to IgE.

Anti-target single domain antibodies may be directed against whole IgE-alpha or a fragment thereof, or a fragment of a homologous sequence thereof.

One embodiment of the present invention is a heterospecific polypeptide construct comprising one or more anti-IgE single domain antibody fused to one or more anti-serum protein single domain antibodies for use in treating, preventing and/or alleviating the symptoms of disorders relating to allergies. Said disorders comprise a wide range of IgE-mediated diseases such as hay fever, asthma, atopic dermatitis, allergic skin reactions, allergic eye reactions and food allergies.

One aspect of the invention is a heterospecific polypeptide construct comprising one or more anti-IgE single domain antibodies fused to one or more anti-serum protein single domain antibodies for use in the treatment, prevention and/or alleviation of disorders relating to allergies, wherein said VHH is administered by inhalation.

Another aspect of the invention is the use of a heterospecific polypeptide construct comprising one or more anti-lgE single domain antibodies fused to one or more anti-serum protein single domain antibodies for the preparation of a medicament for the treatment, prevention and/or alleviation of disorders relating to allergies, wherein said heterospecific polypeptide construct is administered by inhalation.

Another aspect of the invention is a method of treating, preventing and/or alleviating disorders relating to allergies, comprising administering to a subject a heterospecific polypeptide construct comprising one or more anti-IgE single domain antibodies fused to one or more anti-serum protein single domain antibodies by inhalation.

Another aspect of the invention is a heterospecific polypeptide construct comprising one or more anti-IgE single domain antibodies fused to one or more anti-serum protein single domain antibodies for use in the preparation of a medicament for the treatment, prevention and/or alleviation of disorders relating to allergies.

Another aspect of the invention is a use of a heterospecific polypeptide construct comprising one or more anti-IgE single domain antibodies fused to one or more anti-serum protein single domain antibodies for the preparation of a medicament for the treatment, prevention and/or alleviation of disorders relating to allergies.

It is an aspect of the invention that the anti-IgE single domain antibodies of the present invention may be derived from VHHs of any class. For example, they may be derived from a class of VHHs with high homology to the human VH sequence, or may be derived from any of the other classes of VHHs, including the major class of VHH. Said VHHs may be derived from *Camelidae.* These VHHs include the full length *Camelidae* VHHs, domains and may comprise a human Fc domain if effector functions are needed.

The above aspects and embodiments apply to a heterospecific polypeptide construct comprising one or more anti-lgE single domain antibodies fused to one or more anti-serum protein single domain antibodies, wherein the heterospecific polypeptides correspond to a sequence represented by any of SEQ ID NOs: 22 to 24 , a homologous sequence thereof, a functional portion thereof, of a homologous sequence of a functional portion thereof. SEQ ID NOs: 22 to 24 comprise anti-IgE *Camelidae* VHH and anti-mouse serum albumin *Camelidae* VHH.

The above aspects and embodiments apply to a heterospecific polypeptide construct comprising one or more anti-IgE single domain antibodies fused to one or more anti-serum protein single domain antibodies wherein said anti-serum protein single domain antibodies correspond to any of SEQ ID NOs: 1 to 4 (anti-protein serum *Camelidae* VHHs), a homologous sequence thereof, a functional portion thereof, of a homologous sequence of a functional portion thereof.

A heterospecific polypeptide construct as disclosed herein prevents thus reduces or prevents an allergic response due to common or unusual allergens. Furthermore, the construct has a prolonged lifetime in the blood so increasing the therapeutic window.

Interferon gamma (IFN-gamma) is believed to play an important role in various disorders, for example in inflammatory disorders such as rheumatoid arthritis, Crohn's disease, inflammatory bowel disease, ulcerative colitis, multiple sclerosis and hyperimmune reactions in the eye. IFN-gamma has also been shown to play a significant role in the pathology of autoimmune diseases. For example, the presence of IFN-gamma has been implicated in rheumatoid arthritis (Brennan et al, Brit. J. Rheum., 31, 293-8 (1992)) . Several strategies to antagonize the action of these cytokines have been developed and are currently used to treat various disease states.

IFN-gamma in its bioactive form is a dimer and the groove formed by the two subunits is important for its biological activity through interaction with the IFN-gamma receptor. An IFN-gamma inhibitor which has sufficient specificity and selectivity to IFN-gamma may be an efficient prophylactic or therapeutic pharmaceutical compound for preventing or treating inflammatory disorders. Diseases associated with IFN-gamma include multiple sclerosis, rheumatoid arthritis, ankylosing spondylitis, juvenile rheumatoid arthritis, and psoriatic arthritis (US6,333,032 Advanced Biotherapy Concepts, Inc.). Other diseases include Crohn's disease and psoriasis (US6,329,511 Protein Design Labs). Yet other diseases are bowel disease, ulcerative colitis and Crohn's disease (EP0695189 Genentech).

None of the presently available drugs are completely effective for the treatment of autoimmune disease, and most are limited by severe toxicity. In addition, it is extremely difficult and a lengthy process to develop a new chemical entitiy (NCE) with sufficient potency and selectivity to such target sequence. Antibody-based therapeutics on the other hand have significant potential as drugs because they have exquisite specificity to their target and a low inherent toxicity. In addition, the development time can be reduced considerably when compared to the development of new chemical entities (NCE's). However, conventional antibodies are difficult to raise against multimeric proteins where the receptor-binding domain of the ligand is embedded in a groove, as is the case with IFN-gamma.

The heterospecific polypeptide constructs of the present invention, wherein the anti-target single domain antibody is directed against TNF-alpha overcome the problems experienced using peptide therapeutics of the art because of the properties thereof such as stability, size, and reliable expression. Furthermore, the inventors have found that, despite presence of a groove in multimeric IFN-gamma, the heterospecific polypeptide constructs are still able to achieve strong binding to IFNA-gamma.

According to one aspect of the invention, a target against which one or more anti-target single domain antibodies of a heterospecific polypeptide construct comprising one or more anti-target single domain antibodies fused to one or more anti-serum protein single domain antibodies is directed is interferon-gamma (IFN-gamma). IFN-gamma is secreted by some T cells. In addition to its anti-viral activity, IFN-gamma stimulates natural killer (NK) cells and T helper 1 (Th1) cells, and activates macrophages and stimulates the expression of MHC molecules on the surface of cells. Hence, IFN-gamma generally serves to enhance many aspects of immune function, and is a candidate for treatment of disorders where the immune system is over-active *e.g*. Crohn's disease, autoimmune disorders and organ plant rejection in addition inflammatory disorders such as rheumatoid arthritis, Crohn's disease, ulcerative colitis and multiple sclerosis.

One aspect relates to a heterospecific polypeptide construct comprising one or more anti-IFN-gamma single domain antibodies fused to one or more anti-serum protein single domain antibodies, the sequences of said heterospecific polypeptide corresponding to any of SEQ ID NOs: 25 to 27. The anti-IFN-gamma single domain antibodies therein are derived from *Camelidae* heavy chain antibodies (VHHs), which bind to IFN-gamma.

Anti-target single domain antibodies may be directed against whole IFN-gamma or a fragment thereof, or a fragment of a homologous sequence thereof.

One embodiment of the present invention is a heterospecific polypeptide construct comprising one or more anti-IFN-gamma single domain antibodies fused to one or more anti-serum protein single domain antibodies for use in treating, preventing and/or alleviating the symptoms of the disorders wherein the immune system is overactive, as mentioned above. Current therapy consists of intravenous administration of anti-IFN-gamma antibodies. Oral delivery of these heterospecific polypeptide constructs results in the delivery of such molecules in an active form in the colon at sites that are affected by the disorder. These sites are highly inflamed and contain IFN-gamma producing cells. These heterospecific polypeptide constructs can neutralise the IFN-gamma locally, avoiding distribution throughout the whole body and thus limiting negative side-effects. Genetically modified microorganisms such as *Micrococcus lactis* are able to secrete antibody fragments. Such modified microorganisms can be used as vehicles for local production and delivery of antibody fragments in the intestine. By using a strain which produces a IFN-gamma neutralising heterospecific polypeptide construct, inflammatory bowel disorder could be treated.

Another aspect of the invention is a heterospecific polypeptide construct comprising one or more anti-IFN-gamma single domain antibodies fused to one or more anti-serum protein single domain antibodies for use in the treatment, prevention and/or alleviation of disorders wherein the immune system is overactive, wherein said heterospecific polypeptide construct is administered by inhalation.

Another aspect of the invention is the use of a heterospecific polypeptide construct comprising one or more anti-IFN-gamma single domain antibodies fused to one or more anti-serum protein single domain antibodies for the preparation of a medicament for the treatment, prevention and/or alleviation of disorders wherein the immune system is over active, wherein said heterospecific polypeptide construct is administered by inhalation.

Another aspect of the invention is a method of treating, preventing and/or alleviating disorders wherein the immune system is overactive, comprising administering to a subject a heterospecific polypeptide construct comprising one or more anti-IFN-gamma single domain antibodies fused to one or more anti-serum protein single domain antibodies by inhalation.

Another aspect of the invention is a heterospecific polypeptide construct comprising one or more anti-IFN-gamma single domain antibodies joined to one or more anti-serum protein single domain antibodies for use in the preparation of a medicament for the treatment, prevention and/or alleviation of disorders wherein the immune system is overactive.

Another aspect of the invention is a use of a heterospecific polypeptide construct comprising one or more anti-IFN-gamma single domain antibodies fused to one or more anti-serum protein single domain antibodies for use in the preparation of a medicament for the treatment, prevention and/or alleviation of disorders wherein the immune system is over active.

It is an aspect of the invention that the anti-IFN-gamma single domain antibodies of the present invention may be derived from VHHs of any class. For example, they may be derived from a class of VHHs with high homology to the human VH sequence, or may be derived from any of the other classes of VHHs, including the major class of VHH. These VHHs include the full length *Camelidae* VHHs, domains and may comprise a human Fc domain if effector functions are needed.

The above aspect and embodiments apply to a heterospecific polypeptide construct comprising one or more anti-IFN-gamma VHHs fused to one or more anti-serum protein single domain antibodies wherein said heterospecific polypeptide corresponds to a sequence represented by any of SEQ 10 NOs: 25 to 27, a homologous sequence thereof, a functional portion thereof, of a homologous sequence of a functional portion. SEQ ID NOs: 25 to 27 comprise anti-IFN-gamma VHH and anti-mouse serum albumin VHH.

The above aspects and embodiments apply to a heterospecific polypeptide construct comprising one or more anti-IFN-gamma single domain antibodies fused to one or more anti-serum protein VHHs wherein said anti-serum protein VHHs correspond to any of SEQ ID NOs: 1 to 4, a homologous sequence thereof, a functional portion thereof, of a homologous sequence of a functional portion thereof.

One embodiment is a recombinant clone comprising nucleic acid encoding a heterospecific polypeptide construct according to the invention. In one aspect of the invention, said nucleic acid encodes one or more single domain antibodies each directed to a therapeutic or diagnostic target antigen and one or more single domain antibodies directed to a serum protein, said single domain antibodies linked without intervening linkers, or with one or more peptide linker sequences. According to one aspect of the invention, a linker sequence is any suitable linker sequence known in the art. According to another aspect of the invention, a linker sequence is a naturally occurring sequence. Preferred properties of linkers sequences are that they are not immunogenic or not significantly immunogenic, they can provide sufficient flexibility to the heterospecific polypeptide construct, and are resistant to proteolytic degradation. An example of a linker according to the invention is that disclosed in PCT/EP96/01725 which is derived from the hinge region of VHH.

According to another aspect of the invention, a clone comprises nucleic acid encoding a polypeptide corresponding to a sequence represented by any of SEQ ID NOs: 1 to 4, a homologous sequence thereof, a functional portion thereof, or a homologous sequence of a functional portion, and nucleic acid encoding one or more anti-target single domain antibodies, a homologous sequence thereof, a functional portion thereof, or a homologous sequence of a functional portion thereof.

According to another aspect a clone comprises nucleic acid capable of encoding a polypeptide corresponding to a sequence represented by any of SEQ ID NOs:5 to 27, a homologous sequence thereof, a functional portion thereof, or a homologous sequence of a functional portion thereof.

It is within the scope of the invention that nucleic acid encoding multiple anti-target and/or multiple anti-serum VHHs are present in a clone of the invention.

By transforming a compatible host with a clone encoding a heterospecific polypeptide construct of the invention, the heterospecific polypeptide construct can be produced in sufficient quantities for use in therapy. Examples of organisms into which said clone may be transformed include, but are not limited to *E. coli* or *Sacchoromyces cerevisiae.*

Another embodiment of the present invention is a method for prolonging the half-life of an anti-target-VHH comprising the step of joining thereto one or more anti-serum albumin single domain antibodies. As already mentioned above, methods for joining are known in the art or may be any future method, for example, they may be fused by chemical coupling, fused at the DNA level etc.

Treating, preventing and/or alleviating the symptoms of one or more of the disorders mentioned herein generally involves administering to a subject a "therapeutically effective amount" of heterospecific polypeptide construct. By "therapeutically effective amount", "therapeutically effective dose" and "effective amount" means the amount needed to achieve the desired result or results. One of ordinary skill in the art will recognise that the potency and, therefore, an "effective amount" can vary for the various compounds that inhibit a disorder pathway used in the invention. One skilled in the art can readily assess the potency of the compound.

As used herein, the term "compound" refers to a heterospecific polypeptide construct as disclosed herein, a polypeptide represented by SEQ ID NOs: 5 to 27, a homologous sequence thereof, or a homologue thereof, or a nucleic acid capable of encoding said polypeptide.

By "pharmaceutically acceptable" is meant a material that is not biologically or otherwise undesirable, i.e., the material may be administered to an individual along with the compound without causing any undesirable biological effects or interacting in a deleterious manner with any of the other components of the pharmaceutical composition in which it is contained.

The invention disclosed herein is useful for treating or preventing a condition relating to a disorder as mentioned herein (*e.g.* allergy and/or inflammation), in a subject and comprising administering a pharmaceutically effective amount of a compound or composition that binds to a component involved in the disorder pathway (e.g. to IgE and/or TNF-alpha in the blood stream), so inhibiting the disorder pathway and the disorder.

One aspect of the present invention is the use of compounds of the invention for treating or preventing a condition relating to a disorder as mentioned herein (*e.g.* allergy and/or inflammation), in a subject and comprising administering a pharmaceutically effective amount of a compound in combination with another, such as, for example, aspirin.

The present invention is not limited to the administration of formulations comprising a single compound of the invention. It is within the scope of the invention to provide combination treatments wherein a formulation is administered to a patient in need thereof that comprises more than one compound of the invention.

It is well known in the art how to determine the inhibition of a disorder pathway using the standard tests described herein, or using other similar tests. Preferably, the method would result in at least a 10% reduction in an indicator of the disorder, including, for example, 15%, 20%, 25%, 30%, 40%, 50%,60%, 70%, 80%, 90%, 100%, or any amount in between, more preferably by 90%. For example, an inhibition of an allergic pathway by inhibition of IgE by a peptide of the invention might result in a 10% reduction in food-specific IgE levels.

The compound useful in the present invention are formulated as pharmaceutical compositions and administered to a mammalian host, such as a human patient or any animal in a variety of forms adapted to the chosen route of administration, *i,e.*, by inhalation. .

The compound of the present invention can also be administered using gene therapy methods of delivery. See, *e.g.*, U.S. Patent No. 5,399,346, which is incorporated by reference in its entirety. Using a gene therapy method of delivery, primary cells transfected with the gene for the compound of the present invention can additionally be transfected with tissue specific promoters to target specific organs, tissue, grafts, tumors, or cells.

Useful dosages of the compound can be determined by comparing their *in vitro* activity, and *in vivo* activity in animal models. Methods for the extrapolation of effective dosages in mice, and other animals, to humans are known to the art; for example, see U.S. Pat. No. 4,938,949.

Generally, the concentration of the compound(s) in a liquid composition, will be from about 0.1-25 wt-%, preferably from about 0.5-10 wt-%. The concentration in a semisolid or solid composition such as a powder will be about 0.1-5 wt-%, preferably about 0.5-2.5 wt-%.

The amount of the compound, or an active salt or derivative thereof, required for use in treatment will vary not only with the particular salt selected but also with the route of administration, the nature of the condition being treated and the age and condition of the patient and will be ultimately at the discretion of the attendant physician or clinician. Also the dosage of the compound varies depending on the target cell, tumor, tissue, graft, or organ.

The desired dose may conveniently be presented in a single dose or as divided doses administered at appropriate intervals, for example, as two, three, four or more sub-doses per day. The sub-dose itself may be further divided, e.g., into a number of discrete loosely spaced administrations; such as multiple inhalations from an insufflator. .

An administration regimen could include long-term, daily treatment. By "long-term" is meant at least two weeks and preferably, several weeks, months, or years of duration. Necessary modifications in this dosage range may be determined by one of ordinary skill in the art using only routine experimentation given the teachings herein. See Remington's Pharmaceutical Sciences (Martin, E.W., ed. 4), Mack Publishing Co., Easton, PA. The dosage can also be adjusted by the individual physician in the event of any complication.

### EXAMPLES

### Example 1: Immunization of llamas

One Ilama was immunized with human serum albumin (HSA). The immunization scheme is summarized in Table 1.

### Example 2: Repertoire cloning

Peripheral blood lymphocytes (PBLs) were isolated by centrifugation on a density gradient (Ficoll-Paque Plus Amersham Biosciences). PBLs were used to extract total RNA (Chomczynski and Sacchi 1987). cDNA was prepared on 100 µg total RNA with MMLV Reverse Transcriptase (Gibco BRL) using oligo d(T) oligonucleotides. The cDNA was purified with a phenol/chloroform extraction, followed by an ethanol precipitation and subsequently used as template to amplify the VHH repertoire.
In a frst PCR, the repertoire of both conventional (1.6 kb). and heavy-chain (1.3 kb) antibody gene segments were amplified using a leader specific primer (5' - GGCTGAGCTCGGTGGTCCTGGCT- 3') (SEQ ID N° 41) and the oligo d(T) primer (5'-AACTGGAAGAATTCGCGGCCGCAGGAATTTTTTTTTTTTTTTTTT-3') (SEQ ID N° 42). The resulting DNA fragments were separated by agarose gel electrophoresis and the 1.3 kb fragment, encoding heavy-chain antibody segments was purified from the agarose gel. A second PCR was performed using a mixture of FR1 reverse primers and the same oligo d(T) forward primer. The PCR products were digested with *Sfi*I (introduced in the FR1 primer) and *BstE*II (naturally occurring in FR4). Following gel electrophoresis, the DNA fragment of approximately 400 basepairs were purified from gel and ligated into the corresponding restriction sites of phagemid pAX004 to obtain a library of cloned VHHs after etectroporation of *Escherichia coli* TG1. The size of the library was 1.4 x 10⁷ cfu, and all clones contained insert of the correct size.

### Example 3: Rescue of the library, phage preparation

The library was grown at 37°C in 10 ml 2xTY medium containing 2% glucose, and 100 µg/ml ampicillin, until the OD600nm reached 0.5. M13KO7 phages (10¹²) were added and the mixture was incubated at 37°C for 2 x 30 minutes, first without shaking, then with shaking at 100 rpm. Cells were centrifuged for 10 minutes at 4500 rpm at room temperature. The bacterial pellet was resuspended in 50 ml of 2xTY medium containing 100 µg/ml ampicillin and 25 µg/ml kanamycin, and incubated overnight at 37°C with vigorously shaking at 250 rpm. The overnight cultures were centrifuged for 15 minutes at 10,000 rpm at 4°C. Phages were PEG precipitated (20% poly-ethylene-glycol and 1.5 M NaCl) and centrifuged for 30 minutes at 10,000 rpm. The pellet was resuspended in 20 ml PBS. Phages were again PEG precipitated and centrifuged for 30 minutes at 20,000 rpm and 4°C. The pellet was dissolved in 5 ml PBS-1% casein. Phages were titrated by infection of TG1 cells at OD600nm= 0.5 and plating on LB agar plates containing 100 µg/ml ampicillin and 2% glucose. The number of transformants indicates the number of phages (= pfu). The phages were stored at -80°C with 15% glycerol.

### Example 4: Phage ELISA

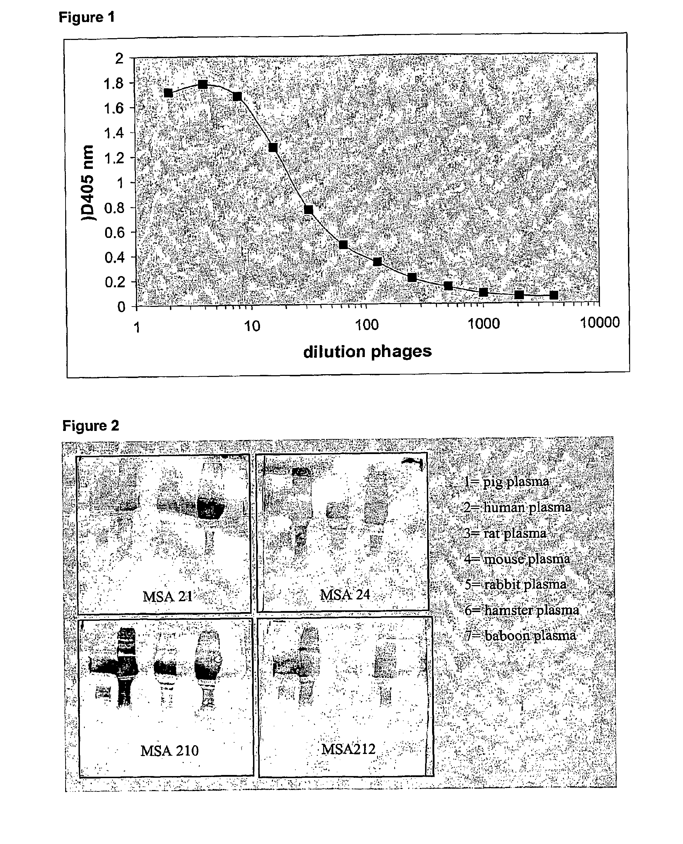

A microtiter plate (Maxisorp) was coated overnight at 4°C with PBS-1 % casein or with 5 µg/ml HSA (human serum albumin). The plate was washed 3 times with PBS-Tween (0.05% Tween20) and blocked for 2 hours at room temperature with 200 µl PBS-1% casein. The plate was washed five times with PBS-Tween. Phages were prepared as described above and applied to the wells in consecutive twofold dilutions. Plates were washed five times with PBS-Tween. Bound phage were detected with a mouse monoclonal antibody anti-M13 conjugated with horse radish peroxidase (HRP) diluted 1/2000 in PBS. The plates were washed five times with PBS-Tween. Staining was performed with ABTS/H₂O₂ and signals were measured after 30 minutes at 405 nm. Results are shown in Figure 1 and indicate the presence of HSA-specific nanobodies in the library.

### Example 5: Selection: first and second round of biopanning

A well in a microtiterplate was coated with 10 µg/ml mouse serum albumin (MSA), or with PBS containing 1% casein. After overnight incubation at 4°C, the wells were blocked with PBS containing 1% casein, for 3 hours at room temperature (RT). 200 µl phages was added to the wells. After 2 hours incubation at RT, the wells were washed 10x with PBS-Tween and 10x with PBS. Bound phages were eluted with 100 µl 0.2 M glycin buffer pH= 2.4. Elutions were performed for 20 minutes at room temperature. Eluted phages were allowed to infect exponentially growing E.Coli TG1 cells, and were then plated on LB agar plates containing 100 µg/ml ampicillin and 2% glucose. A second round was performed with the same conditions as described above. Results are summarized in Table 2.

### Example 6: Screening of individual clones after biopanning

### ELISA: binding to human serum albumin (HSA) and mouse serum albumin (MSA)

A single colony was used to start an overnight culture in LB containing 2% glucose and 100 g/ml ampicillin. This overnight culture was diluted 100-fold in TB medium containing 100 µg/ml ampicillin, and incubated at 37°C until OD600nm= 0.5. 1 mM IPTG was added and the culture was incubated for 3 more hours at 37°C or overnight at 28°C. Cultures were centrifuged for 20 minutes at 10,000 rpm at 4°C. The pellet was frozen overnight or for 1 hour at -20°C. Next, the pellet was thawed at room temperature for 40 minutes, re-suspended in PBS and shaken on ice for 1 hour. Periplasmic fraction was isolated by centrifugation for 20 minutes at 4°C at 20,000 rpm. The supernatant containing the VHH was used for further analysis.
A microtiter plate was coated with 5 µg/ml HSA, with 5 µg/ml mouse serum albumin (MSA) or with PBS-1% casein, overnight at 4°C. Plates were blocked for two hours at room temperature with 300 µl 1% casein in PBS. The plates were washed three times with PBS-Tween. Periplasmic fraction was prepared for 23 individual clones after the first and second round of selection, and allowed to bind to the wells of the microtiterplate. Plates were washed six times with PBS-Tween, after which binding of nanobody was detected by incubation with mouse anti-Histidine monoclonal antibody Serotec MCA 1396 (1/1000 dilution) in PBS for 1 hour at RT followed by anti-mouse-alkaline phosphatase conjugate 1/2000 in PBS, also for 1 hour at RT. Staining was performed with the substrate PNPP (p-nitrophenyl-phosphate, 2 mg/ml in 1 M diethanolamine, 1mM Mg₂SO₄, pH9.8) and the signals were measured after 30 minutes at 405 nm. Results are summarized in Table 3.

### Example 7: Hinfl patern and sequencing

A PCR was performed on positive clones after the second round of panning, with a set of primers binding to a sequence in the vector. The PCR product was digested with the restriction enzyme Hinfl and loaded on a agarose gel. 4 clones were selected with a different Hinfl-pattern for further evaluation. Those clones were sequenced, and results are summarized in Table 4 (SEQ ID NOS: 1, 2 ,3 and 4).

### Example 8: Test cross-reactivity with albumin of different species

A SDS-PAGE was run for plasma (1/10 dilution) from different species (baboon, pig, hamster, human, rat. mouse and rabbit) and blotted on a nitrocellulose membrane. Phages were prepared for clones MSA 21, MSA 24, MSA 210, MSA212 and a control nanobody as described in Example 3. Phages were allowed to bind to the nitrocellulose blotted serum albumins and unbound phages were washed away. Binding was detected with an anti-M13 polyclonal antibody coupled to HRP. DAP was used as a substrate for detection. Results are shown in Figure 2.

From these results we can conclude that all 4 binders are cross-reactive between pig, human, mouse (less for MSA212) and hamster serum albumin. MSA 21 is also cross-reactive with rabbit serum albumin. With the irrelevant nanobody no binding was observed (not shown).

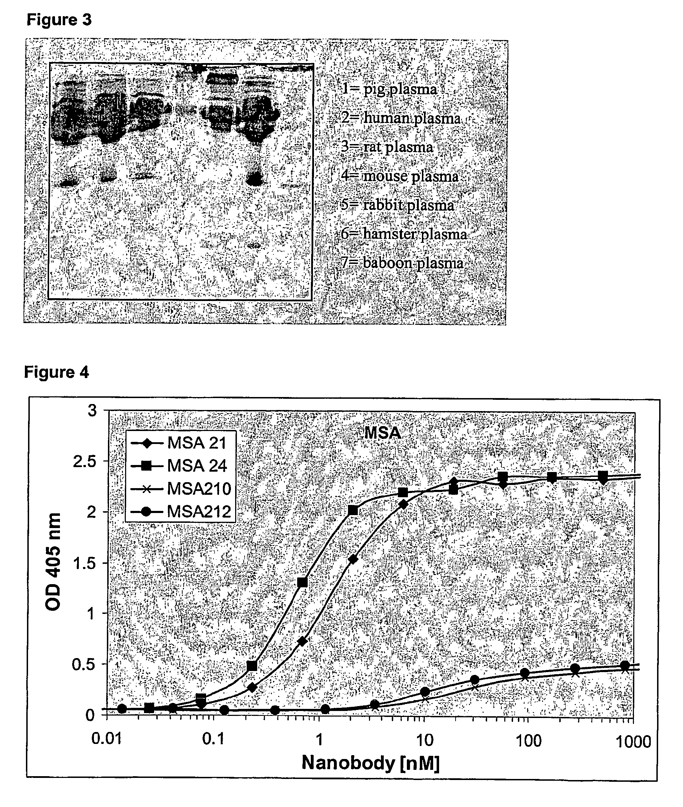

As a control experiment, a SDS-PAGE was run with the different plasma samples diluted 1/100 in PBS. The gel was stained with coomassie. We can conclude from Figure 3 that albumin levels in all plasma samples are high except for rabbit plasma, with low levels of albumin.

### Example 9: Expression and purification

Plasmid was prepared for the binders and was transformed into WK6 electrocompetent cells. A single colony was used to start an overnight culture in LB containing 2% glucose and 100 µg/ml ampicillin. This overnight culture was diluted 100-fold in 300 ml TB medium containing 100 µg/ml ampicillin, and incubated at 37°C until OD600nm= 0.5. 1 mM IPTG was added and the culture was incubated for 3 more hours at 37°C or overnight at 28°C.
Cultures were centrifuged for 20 minutes at 10,000 rpm at 4°C. The pellet was frozen overnight or for 1 hour at -20°C. Next, the pellet was thawed at room temperature for 40 minutes, re-suspended in 20 ml PBS and shaken on ice for 1 hour. Periplasmic fraction was isolated by centrifugation for 20 minutes at 4°C at 20,000 rpm. The supernatant containing the nanobody was loaded on Ni-NTA and purified to homogeneity.

### Example 10: ELISA on MSA of the purified nanobodies

A microtiterplate was coated with 5 µg/ml MSA overnight at 4C. After washing, the plate was blocked for 2 hours at RT with PBS-1 % casein. Samples were applied in duplicate starting at a concentration of 2500 nM at 1/3 dilutions and allowed to bind for 2 hours at RT. A polyclonal rabbit anti-nanobody serum was added at 1/1000 (K208) for one hour at RT. Detection was with anti-rabbit alkaline phosphatase conjugate at 1/1000 and staining with PNPP as described in Example 6. Results are shown in Figure 4.

### Example 11: Construction of bispecific constructs

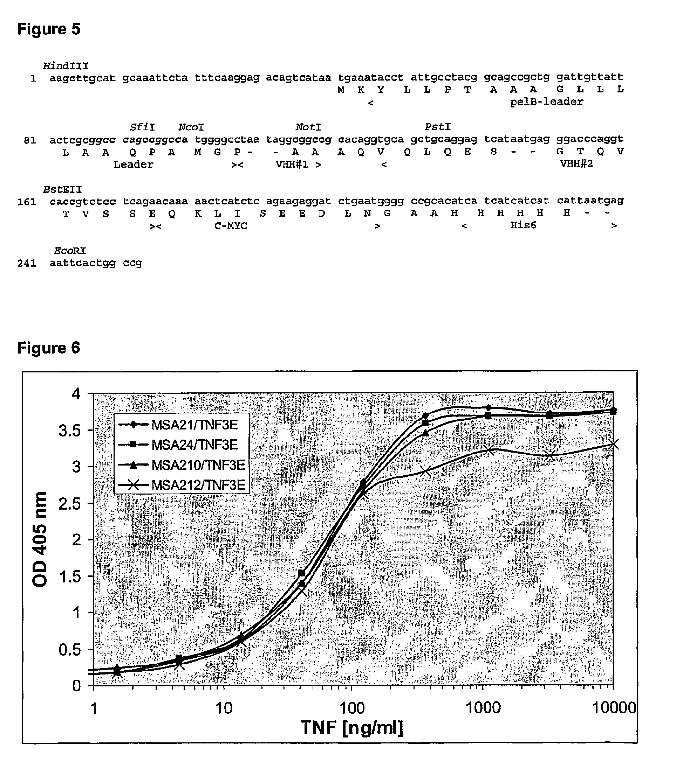

The *E. coli* production vector pAX11 was constructed to allow the two-step cloning of bivalent or bispecific VHH (Figure 5).

The carboxy terminal VHH was cloned first with PstI and BstEII, while in the second step the other VHH was inserted by Sfil and NotI, which do not cut within the first gene fragment. The procedure avoids the enforcement of new sites by amplification and thus the risk of introducing PCR errors. The middle hinge of llama was used as a linker between the nanobodies. A VHH against human TNF alpha was cloned at the COOH terminal of MSA specific nanobodies. Sequences are summarized in Table 4 (SEQ ID NOS: 5, 6, 7 and 8). Plasmid was prepared and was transformed into WK6 electrocompetent cells. A single colony was used to start an overnight culture in LB containing 2% glucose and 100 µg/ml ampicillin. This overnight culture was diluted 100-fold in 300 µl TB medium containing 100 mg/ml ampicillin, and incubated at 37°C until OD600nm= 0.5. 1 mM IPTG was added and the culture was incubated for 3 more hours at 37°C.
Cultures were centrifuged for 20 minutes at 10,000 rpm at 4°C. The pellet was frozen overnight at -20C. The next morning, the pellet was thawed in the cold room for 40 minutes, re-suspended in 20 ml PBS and shaken on ice for 1 hour. Periplasmic fraction was isolated by centrifugation for 20 minutes at 4°C at 10,000 rpm. The supernatant was loaded on Ni-NTA and purified to homogeneity. Sequences are shown in Table 4 (SEQ ID NOS: 5, 6, 7 and 8). A extra purification step was needed to remove some degradation product (5%) on gelfiltration.
Another bispecific VHH against human TNF-alpha (MP7 12b) is listed in Table 4 (SEQ ID NOS: 15, 16,17 and 18).

### Example 12: Test bispecific construct in sandwich ELISA

A microtiter plate was coated with 5 µg/ml MSA overnight at 4°C. Plates were blocked for two hours at room temperature with 300 µl 1% casein in PBS. The plates were washed three times with PBS-Tween. Purified protein for the bispecific constructs was allowed to bind to the wells of the microtiterplate at a concentration of 0.4, 0.5, 2.5 and 2.5 µg/ml for MSA21, MSA24, MSA210 and MSA212 respectively. Plates were washed six times with PBS-Tween, Biotinilated TNF was added at a concentration of 10 µg/ml and diluted 3 fold, and allowed to bind for 2 hours at room temperature. Binding was detected by incubation with mouse extravidin alkaline phosphatase conjugate (Sigma) 1/2000 in PBS, for 1 hour at RT. Staining was performed with the substrate PNPP (p-nitrophenyl-phosphate, 2 mg/ml in 1 M diethanolamine, 1 mM Mg₂SO₄, pH9.8) and the signals were measured after 30 minutes at 405 nm. Results are shown in Figure 6 and indicate that the bispecific construct can bind both antigens simultaneously.

### Example 13: Determine affinity of albumin binders in BIACORE

Affinities for mouse albumin were determined in BIACORE by immobilization of mouse albumin on a CM5 BIAcore chip using EDC-NHS covalent coupling and are summarized in Table 5. The results indicate that the affinity for albumin is retained in the bispecific construct.

### Example 14: Optimization of ELISA in plasma or blood

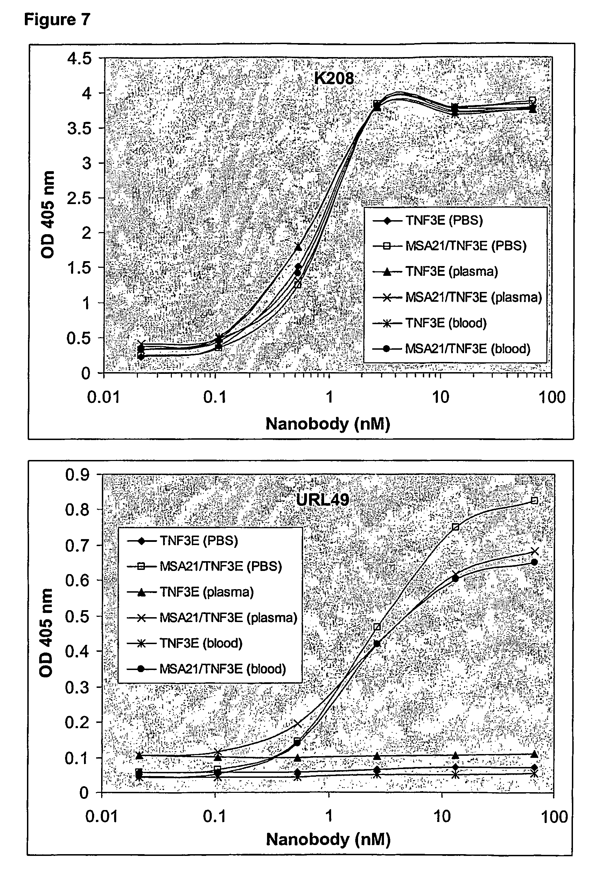

Pharamcokinetic experiments were initiated to compare half life in mice of the TNF-alpha binder TNF3E with MSA21/VHH#3E and MSA24/ VHH#3E. Therefore our ELISA had to be optimized to obtain low background values when the samples are in blood or in plasma. A microtiterplate was coated with neutravidin. After overnight incubation at 4C, the plates were washed and blocked for 2 hours at RT with PBS-1% casein. 1 µg/ml biotinylated TNF-alpha was allowed to bind for 30 minutes at RT and the plate was washed. Samples (monovalent VHH#3E and MSA21/VHH#3E) were applied starting at a concentration of 1 µg/ml, diluted in PBS, 10% plasma or 10% blood and allowed to bind for 2 hours. After washing the plates, a rabbit antiserum was added at a dilution of 1/2000 either recognizing the heavy chain class (K208) or recognizing the conventional class (URL49). After 1 hour incubation, the plates were washed and an anti-rabbit alkaline phosphatase conjugate was added (Sigma) at a dilution of 1/1000. After 1 hour incubation at RT, plates were washed and binding was detected with substrate. Results are shown in Figure 7. The results clearly show that background values with the rabbit antisera (K208 and URL49) are very low when the samples are diluted in 10% blood or 10% plasma as compared to PBS. The URL49 antiserum only recognizes the MSA21/VHH#3E bispecific nanobody and not monovalent VHH#3E, therefore, this antiserum can be used to test the integrity of our bispecific nanobody upon administration to the mice.

### Example 15: Large scale expression and purification of VHH#3E, MSA21NHH#3E and MSA24/VHH#3E for pharmacokinetic studies in mice

3 liter culture was started for monovalent TNF3E and for bispecific MSA21NHH#3E or MSA24NHH#3E and purified as described in Example 11. An extra purification step was needed for the removal of endotoxins. Therefore, samples were purified on a Polymyxin column (BIO-RAD). Samples were analyzed for bacterial endotoxin concentration with the LAL-assay (Limulus Amebocyte Lysate, Bio Whittaker). Results are summarized in Table 6.

### Example 16: Pharmacokinetics in mice

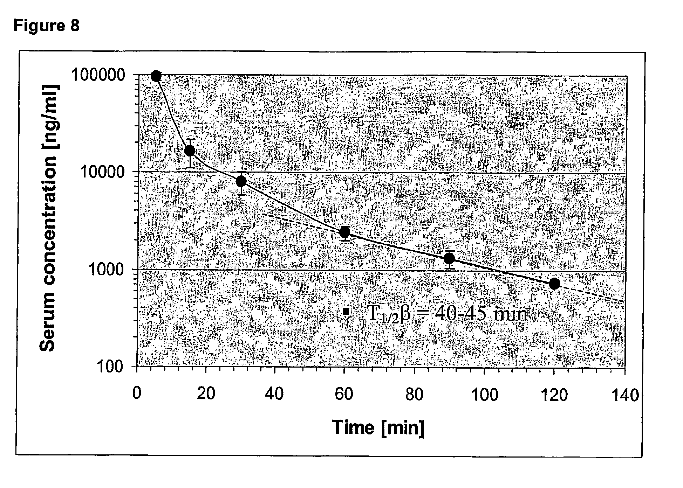

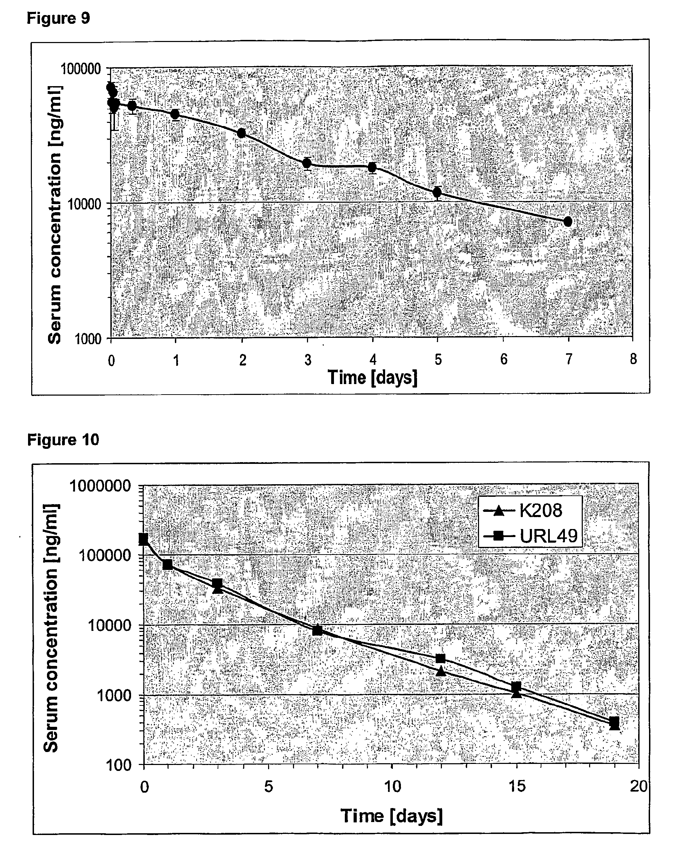

9 mice (CB57/B16) for each construct were injected intravenously in the tail with 100 µg nanobody. Blood was retrieved at different time points (3 mice per time point) and serum was prepared. Samples were analyzed by ELISA for the presence of monovalent or bispecific nanobody as described in example 14. K208 was also compared to URL49 for the bispecific constructs to verify the integrity of the molecule. Results are shown in Figures 8 to 11.

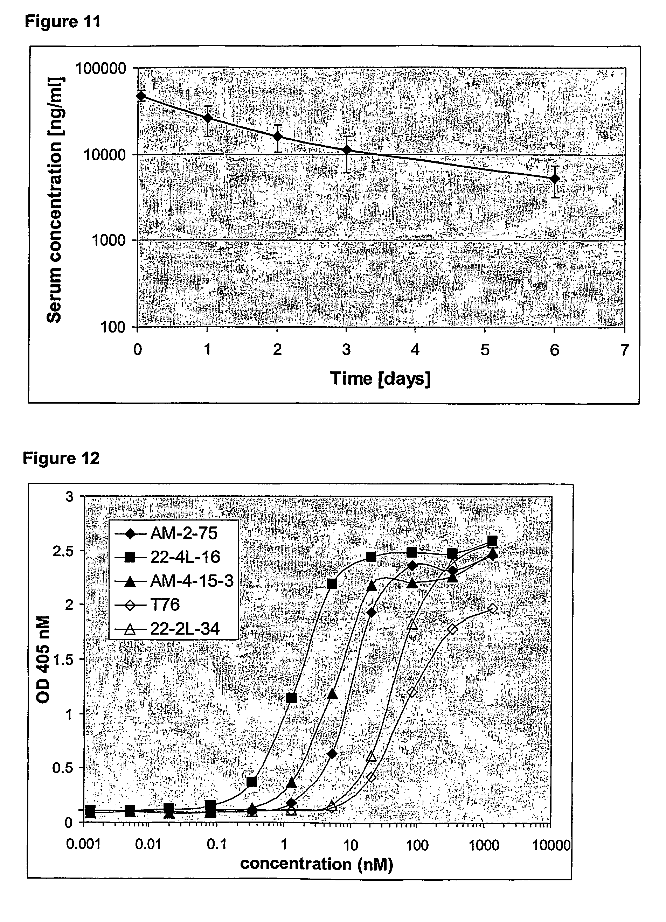

We can conclude from the results that the half life of the monovalent nanobody (40-45 minutes) is dramatically improved by making a bispecific nanobody with specificity for albumin MSA21NHH#3E and MSA24NHH#3E (half-life 2.5 to 3 days). The bispecific nanobody MSA21/VHH#3E remains intact even after 19 days in the mice as shown in ELISA with URL49 (Figure 11).

### Example 17: Further extension of half-life of nanobodies

In order to increase the half-life of MSA21/TNF3E and MSA24/TNF3E even further, a trivalent nanobody was prepared by fusing the bivalent MSA21-MSA21 construct to target-specific nanobody TNF3E. The resulting MSA21/MSA21/TNF3E (Table 7, and SEQ ID NO: 9) was tested *in vivo* according to the method of Example 16.

### Example 18: Immunization of Ilama002

1 Ilama was immunized with vWF. The immunization scheme is summarized in Table 7.

### Example 19: Repertoire cloning and phage preparation

The library was prepared as described in Example 2. The size of the library was 1.4 x 10⁷ cfu, and >90% of the clones contained insert of the correct size. Phages were prepared as described in Example 3.

### Example 20: Selection for binders for vWF inhibiting the interaction with collagen: first and second round of panning

A well in a microtiterplate was coated with 2 µg/ml vWF or with PBS containing 1% casein. After overnight incubation at 4°C, the wells were blocked with PBS containing 1% casein, for 3 hours at RT. 200 µl phages was added to the wells. After 2 hours incubation at RT, the wells were washed 10x with PBS-Tween and 10x with PBS. Phages were specifically eluted with 100 µl of 100 µg/ml collagen type III. Elutions were performed for overnight at room temperature. Eluted phages were allowed to infect exponentially growing TG1 cells, and were then plated on LB agar plates containing 100 µg/ml ampicillin and 2% glucose. This experiment was repeated for a second round of panning, under the same conditions as described above. The results from the panning are presented in Tables 8 and 9.

### Example 21: Functional characterization of vWF binders: Inhibition of binding of vWF to collagen by VHH

A microtiter plate was coated overnight at 4°C with collagen type III at 25 µg/ml in PBS. The plate was washed five times with PBS-Tween and blocked for 2 hours at room temperature with PBS containing 1% casein. The plate was washed five times with PBS-tween. 100 µl of 2 µg/ml vWF (vWF is pre-incubated at 37°C for 15 minutes) was mixed with 20 µl periplasmic extract containing a VHH antibody (described in Example 6) and incubated for 90 minutes at room temperature in the wells of the microtiterplate. The plate was washed five times with PBS-tween. An anti-vWF-HRP monoclonal antibody (DAKO) was diluted 3,000-fold in PBS and incubated for 1 hour. The plate was washed five times with PBS-Tween and vWF-binding was detected with ABTS/H₂O₂. Signals were measured after 30 minutes at 405 nm. The results are presented in Table 10, showing that inhibitors are obtained after the first and second round of panning.

### Example 22: Expression and purification of VHH

Protein was prepared and purified as described in Example 9.

### Example 23: ELISA: binding to vWF

A microtiter plate was coated with 2 µg/ml vWF, overnight at 4°C. Plates were blocked for two hours at room temperature with 300 µl 1% casein in PBS. The plates were washed three times with PBS-Tween. Dilution series of all purified samples were incubated for 2 hours at RT. Plates were washed six times with PBS-Tween, after which binding of VHH was detected by incubation with mouse anti-myc mAB 1/2000 in PBS for 1 hour at RT followed by anti-mouse-HRP conjugate 1/1000 in PBS, also for 1 hour at RT. Staining was performed with the substrate ABTS/H₂O₂ and the signals were measured after 30 minutes at 405 nm. The binding as a function of concentration of purified VHH is indicated in Figure 12.

### Example 24: Inhibition ELISA with purified VHH

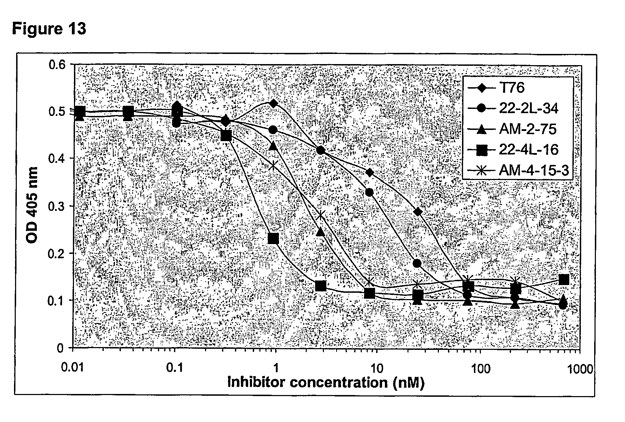

Inhibition ELISA was performed as described in Example 20 but with decreasing concentrations of VHH and with human plasma at a dilution of 1/60 instead of with purified vWF. Results are represented in Figure 13. The concentration of VHH resulting in 50% inhibition (IC50) is given in table 10.

### Example 25: Construction and sequence of bispecific constructs

Bispecific constructs were prepared with the first VHH specific for albumin (MSA21) and the second VHH specific for vWF. Constructs were made as described in Example 11. Sequences are shown in Table 4 (SEQ ID NOS: 19 to 21)

### Example 26: Expression and purification of bispecific constructs

Protein was expressed and purified as described in Example 9. An extra purification step was needed on superdex 75 for removal of some monovalent degradation product (5-10%).

### Example 27: Functionality of both VHHs in the bispecific construct

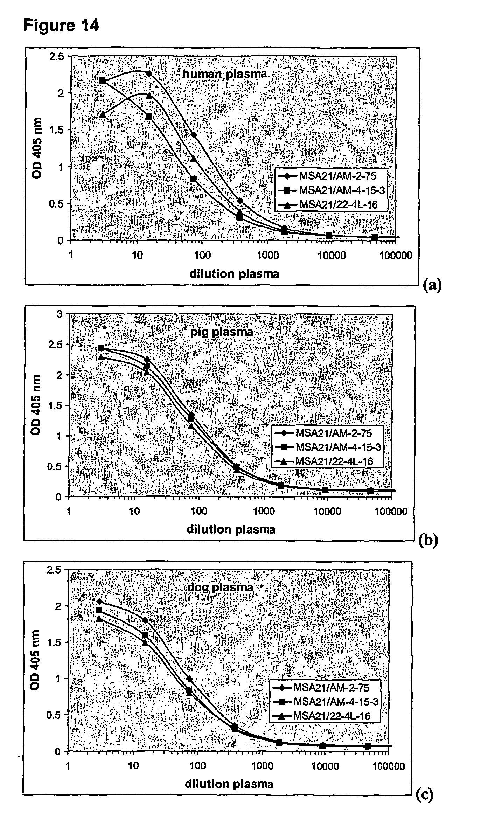

A microtiterplate was coated with 5 µg/ml mouse serum albumin overnight at 4°C. After washing the plate, wells were blocked for 2 hours with PBS-1% casein. The bispecific proteins were allowed to bind to the wells for 2 hours at RT. After washing, human, dog and pig plasma was added at different dilutions and allowed to bind for 2 hours at RT. Binding of vWF was detected with anti-vWF-HRP from DAKO at 1/3000 dilution. Staining was performed with ABTS/H₂O₂. Results are shown in Figure 14 and indicate that functionality of both VHHs is retained in the bispecific construct.

### Example 28: Inhibition of binding of vWF to collagen by the bispecific constructs as compared to the monovalent VHHs

Inhibition for binding of vWF to collagen was tested for monovalent as compared to bispecific constructs as described in Example 20. IC50 values are summarized in Table 11. Results indicate that the inhibitory properties of the VHH are retained in the bispecific construct.

### Example 29: Construction of a bispecific construct containing a VHH-CDR3 fragment fused to an anti-serum albumin VHH

A functional portion, the CDR3 region of MP2F6SR, was amplified by using a sense primer located in the framework 4 region (F6 CRD3 Forward:CTGGCCCCAGAAGTCATACC) (SEQ ID N° 43) and an anti-sense primer located in the framework 3 region (F6 CDR3 Reverse primer:TGTGCATGTGCAGCAAACC) (SEQ ID N° 44).
In order to fuse the CDR-3 fragment with the anti-serum albumin VHH MSA-21, a second round PCR amplification was performed with following primers:
F6 CDR3 Reverse primer Sfi1:
GTCCTCGCAACTGCGGCCCAGCCGGCCTGTGCATGTGCAGCAAACC (SEQ ID N° 45)
F6 CDR3 Forward primer Not1:
GTCCTCGCAACTGCGCGGCCGCCTGGCCCCAGAAGTCATACC (SEQ ID N° 46)

The PCR reactions was performed in 50 ml reaction volume using 50pmol of each primer. The reaction conditions for the primary PCR were 11 min at 94 °C, followed by 30/60/120 sec at 94/55172 °C for 30 cycles, and 5 min at 72°C. All reaction were performed wit 2.5 mM MgCl2, 200 mM dNTP and 1.25U AmpliTaq God DNA Polymerase (Roche Diagnostics, Brussels, Belgium).

After cleavage of the VHH gene of MSA clones with restriction enzymes Pst1/BstEII the digested products were cloned in pAX11 to obtain clones with a VHH at the C-terminus of the multicloning site. The clones were examined by PCR using vector based primers. From clones yielding a 650 bp product, DNA was prepared and used as acceptor vector to clone the CDR3 of MP2F6SR after cleavage of the PCR product with restriction enzymes Sfi1/Not1 to allow N-terminal expression of CDR3 in fusion with a MSA VHH.

### Example 30: Calculation of homologies between anti-target single domain antibodies of the invention

The degree of amino acid sequence homology between anti-target single domain antibodies of the invention was calculated using the Bioedit Sequence Alignment Editor. The calculations indicate the proportion of identical residues between all of the sequences as they are aligned by ClustalW. (Thompson, J.D., Higgins, D.G. and Gibson, T.J. (1994) CLUSTAL W: improving the sensitivity of progressive multiple sequence alignment through sequence weighting, position specific gap penalties and weight matrix choice. Nucleic Acids Research, submitted, June 1994). Table 12 indicates the fraction homology between anti-serum albumin VHHs of the invention. Table 13 indicates the fraction homology between anti-TNF-alpha VHHs of the invention. Table 14 indicates the percentage homology between anti-IFN-gamma VHHs of the invention. Table 15 indicates the fraction homology between anti-vWF VHHs of the invention.

**Table 1: Immunization scheme according to Example 1**

| **Day of immunization** | **HSA** **Llama006** |
|---|---|
| 0 | 100 µg |
| 7 | 100 µg |
| 14 | 50 µg |
| 21 | 50 µg |
| 28 | 50 µg |
| 35 | 50 µg |

**Table 2: results after one and two rounds of panning on mouse serum albumin as described in example 5.**

| | **First round** | **Second round** |
|---|---|---|
| **Pfu mouse serum albumin** | 2.5x10⁷ | 2.5 x 10⁷ |
| **Pfu casein** | 5 x 10³ | 2.5 x 10³ |
| **enrichment** | 5,000 | 10,000 |

**Table 3: Clones were selected after one and two rounds of selection and periplasmic extracts were prepared. These clones were analyzed in ELISA for binding to human and mouse albumin as described in Example 6.**

| | **First round** | **Second round** |
|---|---|---|
| **ELISA mouse serum albumin** | 1/16 | 15/16 |
| **ELISA human serum albumin** | 1/16 | 15/16 |
| **ELISA casein** | 0/16 | 0/16 |

**Table 4: Sequence listing**

| **NAME** | **SEQ ID** | **SEQUENCE** |
|---|---|---|
| | | Anti-mouse serum albumin |
| MSA21 | 1 | QVQLQESGGGLVQPGGSLRLSCEASGFTFSRFGMTWVRQAPGKGVEWVSGISS LGDSTLYADSVKGRFTISRDNAKNTLYLQMNSLKPEDTAVYYCTIGGSLNPGG QGTQVTVSS |
| MSA24 | 2 | QVQLQESGGGLVQPGNSLRLSCAASGFTFRNFGMSWVRQAPGKEPEWVSSISG SGSNTIYADSVKDRFTISRDNAKSTLYLQMNSLKPEDTAVYYCTIGGSLSRSS QGTQVTVSS |
| MSA210 | 3 | QVQLQESGGGLVQPGGSLRLTCTASGFTFSSFGMSWVRQAPGKGLEWVSAISS |
| | | DSGTKNYADSVKGRFTISRDNAKKMLFLQMNSLRPEDTAVYYCVIGRGSPSSQ GTQVTVSS |
| MSA212 | 4 | QVQLQESGGGLVQPGGSLRLTCTASGFTFRSFGMSWVRQAPGKGLEWVSAISA DGSDKRYADSVKGRFTISRDNGKKMLTLDMNSLKPEDTAVYYCVIGRGSPASQ GTQVTVSS |
| MSAc16 | 28 | AVQLVESGGGLVQAGDSLRLSCVVSGTTFSSAAMGWFRQAPGKEREFVGAIKW SGTSTYYTDSVKGRFTISRDNVKNTVYLQMNNLKPEDTGVYTCAADRDRYRDR MGPMTTTDFRFWGQGTQVTVSS |
| MSAcl1 2 | 29 | QVKLEESGGGLVQTGGSLRLSCAASGRTFSSFAMGWFRQAPGREREFVASIGS SGITTNYADSVKGRFTISRDNAKNTVYLQMNSLKPEDTGLCYCAVNRYGIPYR SGTQYQNWGQGTQVTVSS |
| MSAcl1 0 | 30 | EVQLEESGGGLVQPGGSLRLSCAASGLTFNDYAMGWYRQAPGKERDMVATISI GGRTYYADSVKGRFTISRDNAKNTVYLQMNSLKPEDTAIYYCVAHRQTVVRGP YLLWGQGTQVTVSS |
| MSAcl1 4 | 31 | QVQLVESGGKLVQAGGSLRLSCAASGRTFSNYAMGWFRQAPGKEREFVAGSGR SNSYNYYSDSVKGRFTISRDNAKNTVYLQMNSLKPEDTAVYYCAASTNLWPRD RNLYAYWGQGTQVTVSS |
| MSAcl1 6 | 32 | EVQLVESGGGLVQAGDSLRLSCAASGRSLGIYRMGWFRQVPGKEREFVAAISW SGGTTRYLDSVKGRFTISRDSTKNAVYLQMNSLKPEDTAVYYCAVDSSGRLYW TLSTSYDYWGQGTQVTVSS |
| MSAcl1 9 | 33 | QVQLVEFGGGLVQAGDSLRLSCAASGRSLGIYKMAWFRQVPGKEREFVAAISW SGGTTRYIDSVKGRFTLSRDNTKNMVYLQMNSLKPDDTAVYYCAVDSSGRLYW TLSTSYDYWGQGTQVTVSS |
| MSAcl5 | 34 | EVQLVESGGGLVQAGGSLSLSCAASGRTFSPYTMGWFRQAPGKEREFLAGVTW SGSSTFYGDSVKGRFTASRDSAKNTVTLEMNSLNPEDTAVYYCAAAYGGGLYR DPRSYDYWGRGTQVTVSS |
| MScl11 | 35 | AVQLVESGGGLVQAGGSLRLSCAASGFTLDAWPIAWFRQAPGKEREGVSCIRD GTTYYADSVKGRFTISSDNANNTVYLQTNSLKPEDTAVYYCAAPSGPATGSSH TFGIYWNLRDDYDNWGQGTQVTVSS |
| MSAcl1 5 | 36 | EVQLVESGGGLVQAGGSLRLSCAASGFTFDHYTIGWFRQVPGKEREGVSCISS SDGSTYYADSVKGRFTISSDNAKNTVYLQMNTLEPDDTAVYYCAAGGLLLRVE ELQASDYDYWGQGIQVTVSS |
| MSAc18 | 37 | AVQLVDSGGGLVQPGGSLRLSCTASGFTLDYYAIGWFRQAPGKEREGVACISN SDGSTYYGDSVKGRFTISRDNAKTTVYLQMNSLKPEDTAVYYCATADRHYSAS HHPFADFAFNSWGQGTQVTVSS |
| MSAc17 | 38 | EVQLVESGGGLVQAGGSLRLSCAAYGLTFWRAAMAWFRRAPGKERELVVARNW GDGSTRYADSVKGRFTISRDNAKNTVYLQMNSLKPEDTAVYYCAAVRTYGSAT YDIWGQGTQVTVSS |
| MSAc12 0 | 39 | EVQLVESGGGLVQDGGSLRLSCIFSGRTFANYAMGWFRQAPGKEREFVAAINR NGGTTNYADALKGRFTISRDNTKNTAFLQMNSLKPDDTAVYYCAAREWPFSTI PSGWRYWGQGTQVTVSS |
| MSAc14 | 40 | DVQLVESGGGWVQPGGSLRLSCAASGPTASSHAIGWFRQAPGKEREFVVGINR GGVTRDYADSVKGRFAVSRDNVKNTVYLQMNRLKPEDSAIYICAARPEYSFTA MSKGDMDYWGKGTLVTVSS |

| | | Anti-mouse serum albumin/anti TNF-alpha |
|---|---|---|
| MSA21/ VHH#3E | 5 | QVQLQESGGGLVQPGGSLRLSCEASGFTFSRFGMTWVRQAPGKGVEWVSGISS LGDSTLYADSVKGRFTISRDNAKNTLYLQMNSLKPEDTAVYYCTIGGSLNPGG QGTQVTVSSEPKTPKPQPAAAQVQLQESGGGLVQPGGSLRLSCAASGRTFSDHSGYT YTIGWFRQAPGKEREFVARIYWSSGNTYYADSVKGRFAISRDIAKNTVDLTMNNLEPE |
| | | DTAVYYCAARDGIPTSRSVESYNYWGQGTQVTVSS |
| MSA24/ VHH#3E | 6 | QVQLQESGGGLVQPGNSLRLSCAASGFTFRNFGMSWVRQAPGKEPEWVSSISG SGSNTIYADSVKDRFTISRDNAKSTLYLQMNSLKPEDTAVYYCTIGGSLSRSS QGTQVTVSSEPKTPKPQPAAAQVQLQESGGGLVQPGGSLRLSCAASGRTFSDHSGYT YTIGWFRQAPGKEREFVARIYWSSGNTYYADSVKGRFAISRDIAKNTVDLTMNNLEPE DTAVYYCAARDGIPTSRSVESYNYWGQGTQVTVSS |
| MSA210 / VHH#3E | 7 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCAASGRTFSDHSGYTY TIGWFRQAPGKEREFVARIYWSSGNTYYADSVKGRFAISRDIAKNTVDLTMNNLEPED TAVYYCAARDGIPTSRSVESYNYWGQGTQVTVSS |
| MSA212 / VHH#3E | 8 | QVQLQESGGGLVQPGGSLRLTCTASGFTFRSFGMSWVRQAPGKGLEWVSAISA DGSDKRYADSVKGRFTISRDNGKKMLTLDMNSLKPEDTAVYYCVIGRGSPASQ GTQVTVSSEPKTPKPQPAAAQVQLQESGGGLVQPGGSLRLSCAASGRTFSDHSGYTY TIGWFRQAPGKEREFVARIYWSSGNTYYADSVKGRFAISRDIAKNTVDLTMNNLEPED TAVYYCAARDGIPTSRSVESYNYWGQGTQVTVSS |
| MSA21/ MSA21/ VHH#3E | 9 | QVQLQESGGGLVQPGGSLRLSCEASGFTFSRFGMTWVRQAPGKGVEWVSGISS LGDSTLYADSVKGRFTISRDNAKNTLYLQMNSLKPEDTAVYYCTIGGSLNPGG QGTQVTVSSEPKTPKPQPAAAQVQLQESGGGLVQPGGSLRLSCEASGFTFSRF GMTWVRQAPGKGVEWVSGISSLGDSTLYADSVKGRFTISRDNAKNTLYLQMNS LKPEDTAVYYCTIGGSLNPGGQGTQVTVSSEPKTPKPQPAAAQVQLQESGGGL VQPGGSLRLSCAASGRTFSDHSGYTYTIGWFRQAPGKEREFVARIYWSSGNTY YADSVKGRFAISRDIAKNTVDLTMNNLEPEDTAVYYCAARDGIPTSRSVESYN YWGQGTQVTVSS |
| MSA210 /VHH#1 | 10 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCATSGFDFSVSW MYWVRQAPGKGLEWVSEINTNGLITKYVDSVKGRFTISRDNAKNTLYLQMDSL IPEDTALYYCARSPSGSFRGQGTQVTVSS |
| MSA210 /VHH#9 | 11 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCAASGSIFRVNA MGWYRQVPGNQREFVAIITSGDNLNYADAVKGRFTISTDNVKKTVYLQMNVLK PEDTAVYYCNAILQTSRWSIPSNYWGQGTQVTVSS |
| MSA210 /VHH#1 3 | 12 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCATSGFTFSDYW MYWVRQAPGKGLEWVSTVNTNGLITRYADSVKGRFTISRDNAKYTLYLQMNSL KSEDTAVYYCTKVVPPYSDDSRTNADWGQGTQVTVSS |
| MSA210 /VHH#2 | 13 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCAASGRTFSDHS GYTYTIGWFRQAPGKEREFVARIYWSSGNTYYADSVKGRFAISRDIAKNTVDL TMNNLEPEDTAVYYCAARDGIPTSRSVESYNYWGQGTQVTVSS |
| MSA210 /VHH#3 | 14 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQDSGGGLVQAGGSLRLSCAVSGRTFSAHS VYTMGWFRQAPGKEREFVARIYWSSANTYYADSVKGRFTISRDNAKNTVDLLM NSLKPEDTAVYYCAARDGIPTSRTVGSYNYWGQGTQVTVSS |
| MSA21/ VHH#12 | 15 | QVQLQESGGGLVQPGGSLRLSCEASGFTFSRFGMTWVRQAPGKGVEWVSGISS LGDSTLYADSVKGRFTISRDNAKNTLYLQMNSLKPEDTAVYYCTIGGSLNPGG |
| B | | QGTQVTVSSEPKTPKPQPAAAQVQLQESGGGLVQPGGSLRLSCAASGFEFENH WMYWVRQAPGKGLEWVSTVNTNGLITRYADSVKGRFTISRDNAKYTLYLQMNS LKSEDTAVYYCTKVLPPYSDDSRTNADWGQGTQVTVSS |
| MSA24/ VHH#12 B | 16 | QVQLQESGGGLVQPGNSLRLSCAASGFTFRNFGMSWVRQAPGKEPEWVSSISG SGSNTIYADSVKDRFTISRDNAKSTLYLQMNSLKPEDTAVYYCTIGGSLSRSS QGTQVTVSSEPKTPKPQPAAAQVQLQESGGGLVQPGGSLRLSCAASGFEFENH WMYWVRQAPGKGLEWVSTVNTNGLITRYADSVKGRFTISRDNAKYTLYLQMNS LKSEDTAVYYCTKVLPPYSDDSRTNADWGQGTQVTVSS |
| MSA210 /VHH#1 2B | 17 | QVQLQESGGGLVQPGGSLRLTCTASGFTFSSFGMSWVRQAPGKGLEWVSAISS DSGTKNYADSVKGRFTISRDNAKKMLFLQMNSLRPEDTAVYYCVIGRGSPSSQ GTQVTVSSEPKTPKPQPAAAQVQLQESGGGLVQPGGSLRLSCAASGFEFENHW MYWVRQAPGKGLEWVSTVNTNGLITRYADSVKGRFTISRDNAKYTLYLQMNSL KSEDTAVYYCTKVLPPYSDDSRTNADWGQGTQVTVSS |
| MSA212 /VHH#1 2B | 18 | QVQLQESGGGLVQPGGSLRLTCTASGFTFRSFGMSWVRQAPGKGLEWVSAISA DGSDKRYADSVKGRFTISRDNGKKMLTLDMNSLKPEDTAVYYCVIGRGSPASQ GTQVTVSSEPKTPKPQPAAAQVQLQESGGGLVQPGGSLRLSCAASGFEFENHW MYWVRQAPGKGLEWVSTVNTNGLITRYADSVKGRFTISRDNAKYTLYLQMNSL KSEDTAVYYCTKVLPPYSDDSRTNADWGQGTQVTVSS |
| | | |

| | | Anti-mouse serum albumin/anti-vWF |
|---|---|---|
| MSA21/A M-2-75 | 19 | QVQLQESGGGLVQPGGSLRLSCEASGFTFSRFGMTWVRQAPGKGVEWVSGIS SLGDSTLYADSVKGRFTSRDNAKNTLYLQMNSLKPEDTAVYYCTIGGSLNPG GQGTQVTVSSEPKTPKPQPAAAQVQLQESGGGLVQPGGSLRLSCAASGFNFN WYPMSWVRQAPGKGLEWVSTISTYGEPRYADSVKADSPSSETTPTTRCICNE QPETEDTAVYYCARGAGTSSYLPQRGNWDQGTQVTVSS |
| MSA21/A M-4-15-3 | 20 | QVQLQESGGGLVQPGGSLRLSCEASGFTFSRFGMTWVRQAPGKGVEWVSGIS SLGDSTLYADSVKGRFTSRDNAKNTLYLQMNSLKPEDTAVYYCTIGGSLNPG GQGTQVTVSSEPKTPKPQPAAAQVQLQDSGGGLVQPGGSLRLACAASGSIFS INSMGWYRQAPGKQRELVAHALADGSASYRDSVKGRFTISRDNAKNTVYLQM NSLKPEDTAVYYCNTVPSSVTKGYWGQGTQVTVSS |
| MSA21/2 2-4L-16 | 21 | QVQLQESGGGLVQPGGSLRLSCEASGFTFSRFGMTWVRQAPGKGVEWVSGIS SLGDSTLYADSVKGRFTSRDNAKNTLYLQMNSLKPEDTAVYYCTIGGSLNPG GQGTQVTVSSEPKTPKPQPAAAQVQLVESGGGLVQAGGSLRLSCAASGRTFS SYAMGWFRQAPGKEREFVAAISWSGGSTYYADSVKGRFTISRDNAKNTVYLQ MNSLKPEDTAVYYCVADTGGISWIRTQGYNYWGQGTQVTVSS |

| | | Anti-mouse serum albumin/anti-IgE |
|---|---|---|
| MSA 21/ EV 2H11 | 22 | QVQLQESGGGLVQPGGSLRLSCEASGFTFSRFGMTWVRQAPGKGVEW VSGISSLGDSTLYADSVKGRFTISRDNAKNTLYLQMNSLKPEDTAVY YCTIGGSLNPGGQGTQVTVSSEPKTPKPQPAAAQVQLQESGGGLVQA GGSLRLSCAASGVTFSSYAMGWFRQAPGKEREFVASITWTGTGTYYA DSVKGRFTISRDHAGTTVYLQMNSLKPEDTAVYYCAVDRRSSTYYLM KGEYDYRGRGTQVTVSS |
| MSA 24/ EV 2H11 | 23 | QVQLQESGGGLVQPGNSLRLSCAASGFTFRNFGMSWVRQAPGKEPEW VSSISGSGSNTIYADSVKDRFTISRDNAKSTLYLQMNSLKPEDTAVY YCTIGGSLSRSSQGTQVTVSSEPKTPKPQPAAAQVQLQESGGGLVQA GGSLRLSCAASGVTFSSYAMGWFRQAPGKEREFVASITWTGTGTYYA DSVKGRFTISRDHAGTTVYLQMNSLKPEDTAVYYCAVDRRSSTYYLM KGEYDYRGRGTQVTVSS |
| MSA | 24 | QVQLQESGGGLVQPGGSLRLTCTASGFTFSSFGMSWVRQAPGKGLEW |
| 210/ EV 2H11 | | VSAISSDSGTKNYADSVKGRFTISRDNAKKMLFLQMNSLRPEDTAVY YCVIGRGSPSSQGTQVTVSSEPKTPKPQPAAAQVQLQESGGGLVQAG GSLRLSCAASGVTFSSYAMGWFRQAPGKEREFVASITWTGTGTYYAD SVKGRFTISRDHAGTTVYLQMNSLKPEDTAVYYCAVDRRSSTYYLMK GEYDYRGRGTQVTVSS |

| | | Anti-mouse serum albumin/anti-IFN-aamma |
|---|---|---|
| MSA 21/ MP2F6SR | 25 | QVQLQESGGGLVQPGGSLRLSCEASGFTFSRFGMTWVRQAPGKGVEW VSGISSLGDSTLYADSVKGRFTISRDNAKNTLYLQMNSLKPEDTAVY YCTIGGSLNPGGQGTQVTVSSEPKTPKPQPAAAQVKLEESGGGLVQA GGSLRLSCAASGRTFNNYNMGWFRQAPGKEREFVAAISWNGGSTYYD DSVKGRFTISRDNANNLVYLQMNSLNFEDTAVYYCACAANPYGIPQY RENRYDFWGQGTQVTVSS |
| MSA 24/ MP2F1BR | 26 | QVQLQESGGGLVQPGNSLRLSCAASGFTFRNFGMSWVRQAPGKEPEW VSSISGSGSNTIYADSVKDRFTISRDNAKSTLYLQMNSLKPEDTAVY YCTIGGSLSRSSQGTQVTVSSEPKTPKPQPAAAAVQLVESGGGLVQT GDSLRLSCVASGGTFSRYAMGWFRQAPGKEREFVARIGYSGRSISYA TSVEGRFAISRDNAKNTVYLQMNSLKPEDTAVYYCASLVSGTLYQAD YWGQGTQVTVSS |
| MSA 210/ MP3H6SR A | 27 | QVQLQESGGGLVQPGGSLRLTCTASGFTFSSFGMSWVRQAPGKGLEW VSAISSDSGTKNYADSVKGRFTISRDNAKKMLFLQMNSLRPEDTAVY YCVIGRGSPSSQGTQVTVSSEPKTPKPQPAAAQVQLQESGGGLVQAG GSLRLSCAASGRTFSIYNMGWFRQAPGKEREFVAGISWNGGSIYYTS SVEGRFTISRDNAENTVYLQMNSLKPEDTGVYYCASKGRPYGVPSPR QGDYDYWGQGT QVTVSS |

**Table 5: Affinities (koff, kon and KD) for albumin binders as determined by BIACORE as described in Example 13.**

| | **Kₒₙ (10⁵ M⁻¹S⁻¹)** | **K_{off} (10⁻⁵s⁻¹)** | **K_{D} [nM]** |
|---|---|---|---|
| **MSA21** | 3.4 | 420 | 12 |
| **MSA24** | 6.4 | 1800 | 28 |
| **MSA212** | 3.7 | 9330 | 250 |
| **MSA21/TNF3E** | 2.3 | 370 | 16 |
| **MSA24/TNF3E** | 3.1 | 630 | 20 |
| **MSA212/TNF3E** | 0.42 | 490 | 120 |

**Table 6: Results for the LAL-assay for monovalent and bispecific nanobodies after purification on polymyxin as described in Example 15.**

| | **Monovalent TNF3E** | **Bispecific MSA21/TNF3E** | **Bispecific MSA24/TNF3E** |
|---|---|---|---|
| **Endotoxin units/mg of VHH** | 0.13 Eu/mg | 0.75 Eu/mg | 2.8 Eu/mg |

**Table 7: Immunization scheme used for llama 002 according to Example 17.**

| **Llama002 Day of immunization** | **vWF** |
|---|---|
| 0 | 100 µg |
| 7 | 100 µg |
| 14 | 50 µg |
| 21 | 50 µg |
| 28 | 50 µg |
| 35 | 50 µg |

**Table 8: Plaque forming units (pfu) after one or two round(s) of panning on vWF as compared to PBS-casein as described in example 19. Pfu vWF (antigen) divided by pfu casein (a specific binding) = enrichment.**

| **round** | **Pfu vWF** | **Pfu casein** | **Enrichment** |
|---|---|---|---|
| First | 1 x 10⁷ | 2.5 x 10⁵ | 40 |
| Second | 5 x 10⁸ | 2.5 x 10⁶ | 200 |

**Table 9: Number of inhibitors versus the number of clones tested after the first and the second round of panning as described in Example 20.**

| **round** | **Number of inhibitors versus number of clones tested** |
|---|---|
| First | 4/800 |
| Second | 4/96 |

**Table 10: concentration of VHH (nM) needed to inhibit binding of vWF to collagen by 50% (IC50) as described in Example 23.**

| **Name VHH** | **IC50 (nM)** |
|---|---|
| **22-2L-34** | 10 |
| **T76** | 30 |
| **AM-4-15-3** | 2 |
| **22-4L-16** | 0.5 |
| **C37** | 2 |
| **AM-2-75** | 2 |

**Table 11: IC50 values for bispecific nanobodies against albumin and against vWF as described in Example 28.**

| | **IC50 (ng/ml)** |
|---|---|
| **AM-2-75** | 100 |
| **MSA21/AM-2-75** | 60 |
| **AM-4-15-3** | 155 |
| **MSA21/AM-4-15-3** | 245 |
| **22-4L-16** | 100 |
| **MSA21/22-4L-16** | 140 |

**Table 12: Fractional homologies between the amino acid sequences of anti-mouse serum albumin VHHs of the invention.**

| **SEQ** | **MSA21** | **MSA24** | **MSA210** | **MSA212** |
|---|---|---|---|---|
| **MSA21** | 1.000 | 0.834 | 0.800 | 0.782 |
| **MSA24** | -- | 1.000 | 0.782 | 0.791 |
| **MSA210** | --- | --- | 1.000 | 0.903 |
| **MSA212** | --- | --- | -- | 1.000 |

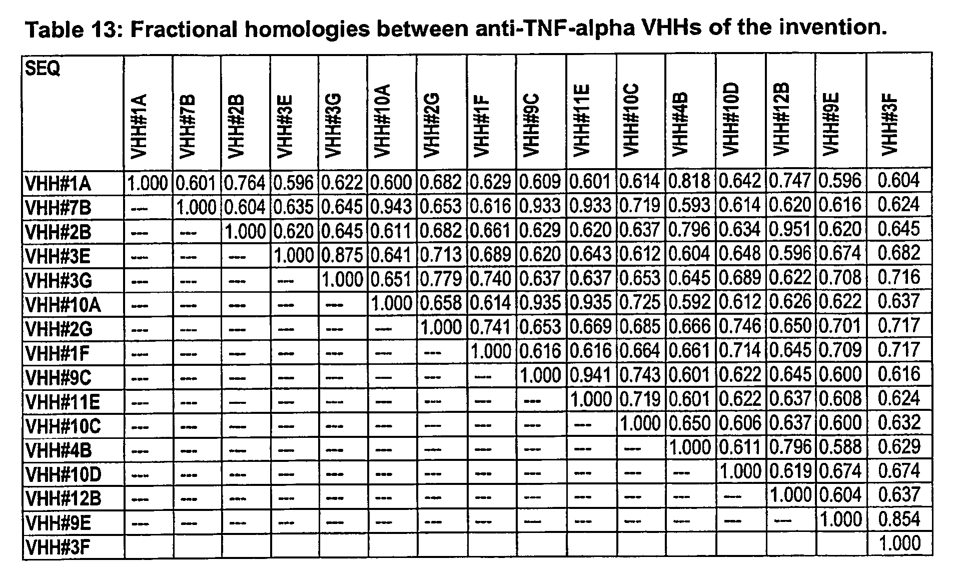

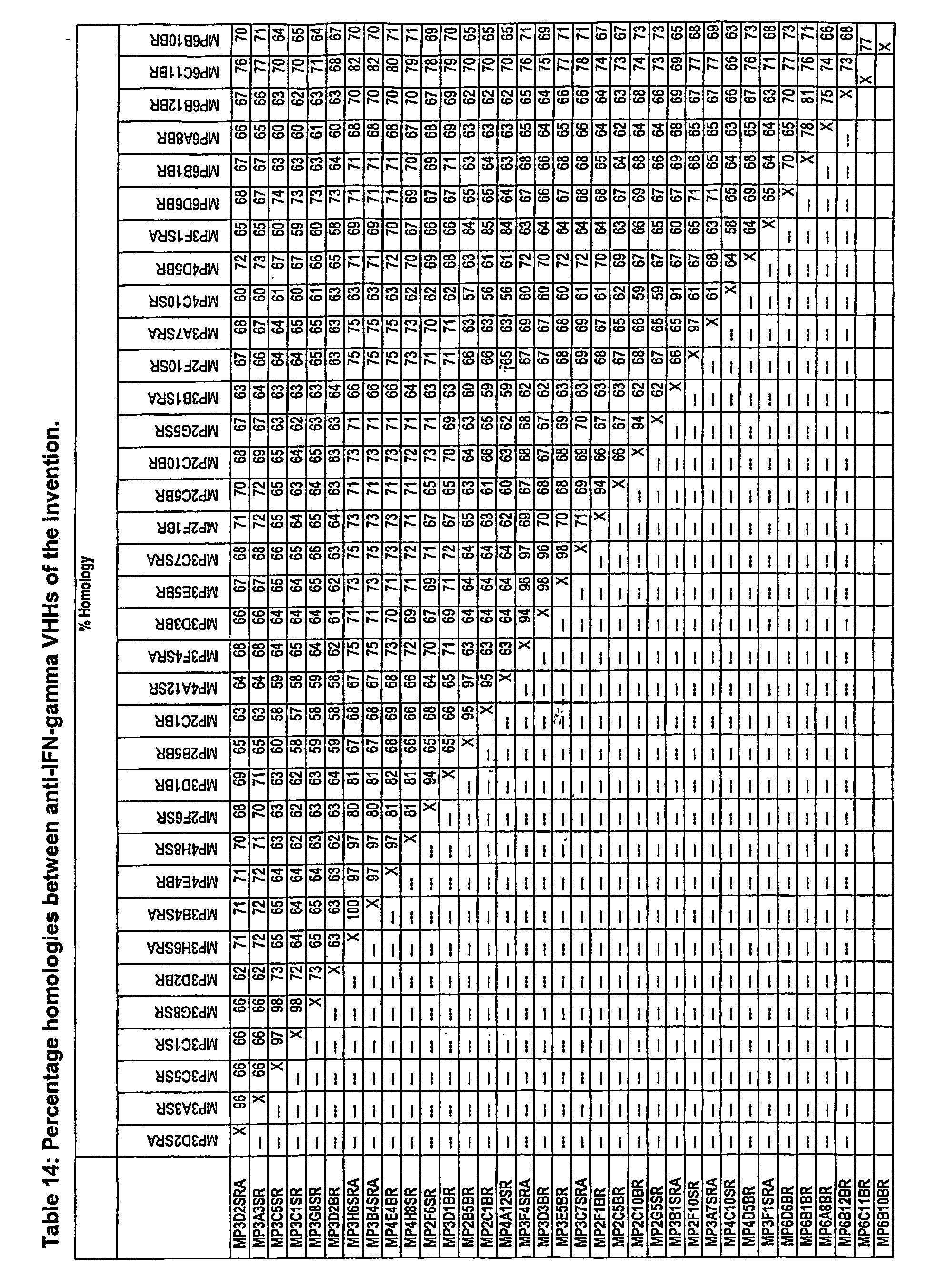

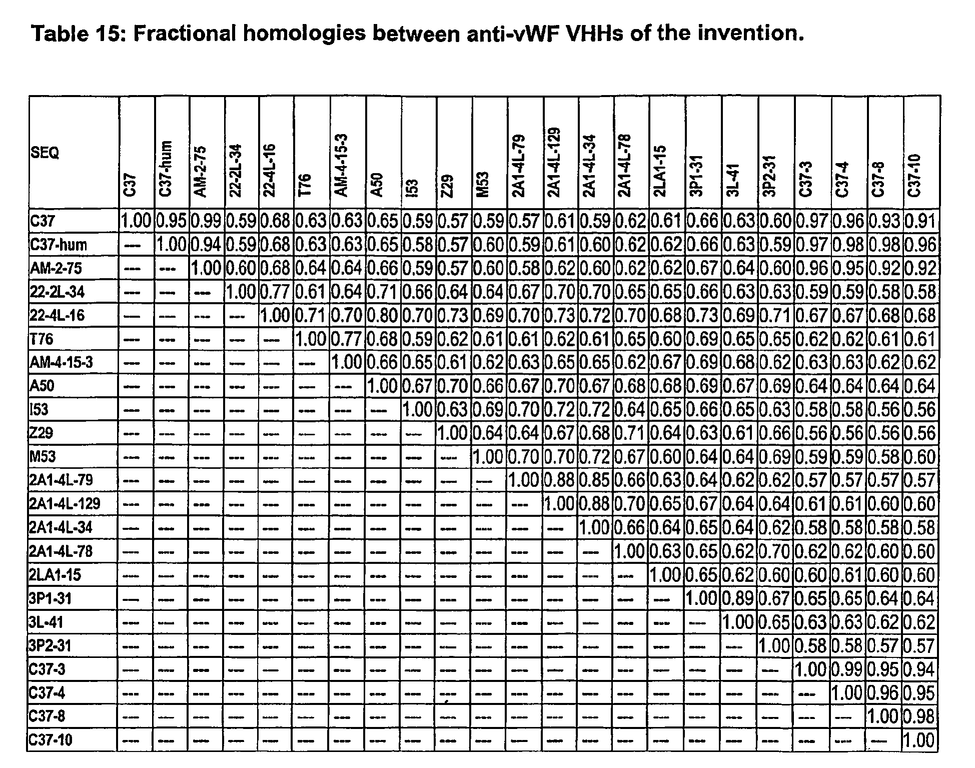

## Claims

1. Pharmaceutical composition for use in therapy comprising:
- one or more Camelidae VHH or humanized Camelidae VHH each directed against a serum protein of a subject, and
- one or more single domain antibodies each directed against a therapeutic and/or diagnostic target
and a pharmaceutically acceptable vehicle
**characterized in that** it is administered by inhalation.

2. Pharmaceutical composition according to claim 1, wherein the single domain antibodies directed against a target molecule are anti-IgE single domain antibodies.

3. Pharmaceutical composition according to any of claims 1 or 2, which is formulated in the for form a nasal spray (e.g. aerosol), inhaler, pump-type sprayer or aerosol sprayer.

4. Pharmaceutical composition according to any of claims 1 to 3, which is a lyophilized VHH formulation.

5. Pharmaceutical composition according to any of claims 1 to 4, comprising a solid carrier.

6. Pharmaceutical composition according to claim 5, wherein the solid carrier is selected from talc, clay, microcrystalline cellulose, silica and alumina.

7. Pharmaceutical composition according to any of claims 1 to 4, comprising a liquid carrier.

8. Pharmaceutical composition according to claim 7, wherein the liquid carrier is selected from water, hydroxyalkyls, glycols and water-alchol/glycol blends.

9. Pharmaceutical composition according to any of claims 1 to 8, wherein the single domain antibodies are single domain antibodies derived from a conventional 4-chain antibody (VH) or derived from a heavy chain antibody naturally devoid of light chain (VHH).

10. Pharmaceutical composition according to any of claims 1 to 9, wherein the single domain antibody is humanized.

11. Pharmaceutical composition according to any of claims 1 to 10, wherein
- the number of anti-target single domain antibodies is at least two, and
- at least two anti-target single domain antibodies do not share the same sequence, or all anti-target single domain antibodies share the same sequence.

12. Pharmaceutical composition according to claim 11, wherein the single domain antibodies that are directed against a target have the same sequence or wherein at least two anti-target single domain antibodies have a different sequence but are directed against the same epitopes or different epitopes on the same target.

13. Pharmaceutical composition according to any of claims 1 to 12, wherein
- the number of anti-serum protein single domain antibodies is at least two, and
- at least two anti-serum protein single domain antibodies do not share the same sequence, or all anti-serum protein single domain antibodies share the same sequence.

14. Pharmaceutical composition according to claims 13, wherein the single domain antibodies that are directed against a serum protein have the same sequence or wherein at least two anti-serum protein single domain antibodies have a different sequence but are directed against the same epitopes or different epitopes on the same serum protein.

15. Pharmaceutical composition according to any of claims 1 to 14, wherein the serum protein is selected from serum albumin, serum immunoglobulins, thyroxine-binding protein, transferrin and, fibrinogen or fragment thereof.

16. Pharmaceutical composition according to any of claims 1 to 15, wherein the anti-serum protein single domain antibody corresponds to a sequence represented by any of SEQ ID NOs: 1 to 4 and 28 to 40; or
a) an homologous sequence of any of SEQ ID NOs: 1 to 4 and 28 to 40, comprising 1, 2, 3, 5, 6, 7, 8, 10 additions, deletions or substitutions of one or more amino acids, which do not substantially alter the functional characteristics of the single domain antibody;
b) an homologous sequence of any of SEQ ID NOs: 1 to 4 and 28 to 40, with a sequence identity of more than 70%, 75%, 80%, 85%, 90%, 95% or 98% with SEQ ID NO: 1 to 4 and 28 to 40;
c) a functional portion of any of SEQ ID NOs: 1 to 4 and 28 to 40 that maintains the intereaction with the target with affinity of 10⁻⁶ M or better;
d) a functional portion of any of SEQ ID NOs: 1 to 4 and 28 to 40, that comprises a partial deletion of the complete amino acid sequence and still maintains the binding site(s) and protein domain(s) necessary for the binding of and interaction with the target.

17. Pharmaceutical composition according to any of claims 1 to 16, wherein the heterospecific polypeptide construct is represented by any of SEQ ID NOs: 22 to 24; or
a) an homologous sequence of any of SEQ ID NOs: 22 to 24 comprising 1, 2, 3, 5, 6, 7, 8, 10 additions, deletions or substitutions of one or more amino acids, which do not substantially alter the functional characteristics of the heterospecific polypeptide;
b) an homologous sequence of any of SEQ ID NOs: 22 to 24 with a sequence identity of more than 70%, 75%, 80%, 85%, 90%, 95% or 98% with SEQ ID NO: 22 to 24;
c) a functional portion of any of SEQ ID NOs: 22 to 24 that maintains the intereaction with the target with affinity of 10⁻⁶ M or better;
d) a functional portion of any of SEQ ID NOs: 22 to 24 that comprises a partial deletion of the complete amino acid sequence and still maintains the binding site(s) and protein domain(s) necessary for the binding of and interaction with the target.

18. Pharmaceutical composition according to any of claims 1 to 17, for treating, preventing and/or alleviating the symptoms of a disorder relating to allergies.

19. Pharmaceutical composition according to claim 18, wherein said disorder is selected from hay fever, asthma, atopic dermatitis, allergic skin reaction, allergic eye reactions and food allergies.

## Patentansprüche

1. Pharmazeutische Zusammensetzung zur Verwendung in der Therapie, umfassend:
- eine oder mehrere Cameliden-VHH oder eine humanisierte Cameliden-VHH, jedes davon gegen ein Serumprotein eines Individuums gerichtet, sowie
- einen oder mehre Einzeldomänenantikörper, jeder gegen ein therapeutisches und/oder diagnostisches Target gerichtet
und einen pharmazeutisch annehmbaren Träger,
**dadurch gekennzeichnet, dass** sie durch Inhalation verabreicht wird.

2. Pharmazeutische Zusammensetzung gemäß Anspruch 1, wobei die Einzeldomänenantikörper, die gegen ein Targetmolekül gerichtet sind, Anti-IgE-Einzeldomänenantikörper sind.

3. Pharmazeutische Zusammensetzung gemäß einem der Ansprüche 1 oder 2, die in der Form eines Nasensprays (z.B. Aerosol), Inhalationsgerätes, Pumpensprayers oder Aerosolsprayers formuliert ist.

4. Pharmazeutische Zusammensetzung gemäß einem der Ansprüche 1 bis 3, die eine lyophilisierte VHH-Formulierung ist.

5. Pharmazeutische Zusammensetzung gemäß einem der Ansprüche 1 bis 4, die einen festen Träger umfasst.

6. Pharmazeutische Zusammensetzung gemäß Anspruch 5, wobei der feste Träger aus Talk, Lehm, mikrokristalliner Zellulose, Siliciumoxid und Aluminiumoxid ausgewählt wird.

7. Pharmazeutische Zusammensetzung gemäß einem der Ansprüche 1 bis 4, die einen flüssigen Träger umfasst.

8. Pharmazeutische Zusammensetzung gemäß Anspruch 7, wobei der flüssige Träger ausgewählt wird aus Wasser, Hydroxyalkylverbindungen, Glycolen und Wasser-Alkohol/Glycolmischungen.

9. Pharmazeutische Zusammensetzung gemäß einem der Ansprüche 1 bis 8, wobei die Einzeldomänenantikörper Einzeldomänenantikörper sind, die von einem herkömmlichen vierkettigen Antikörper (VH) oder von der schweren Kette eines Antikörpers, dem natürlicherweise die leichten Ketten fehlen (VHH), abgeleitet sind.

10. Pharmazeutische Zusammensetzung gemäß einem der Ansprüche 1 bis 9, wobei der Einzeldomänenantikörper humanisiert ist.

11. Pharmazeutische Zusammensetzung gemäß einem der Ansprüche 1 bis 10, wobei
- die Anzahl der Anti-Target-Einzeldomänenantikörper mindestens zwei ist, sowie
- mindestens zwei Anti-Target-Einzeldomänenantikörper nicht die gleiche Sequenz teilen, oder alle Anti-Target-Einzeldomänenantikörper die gleiche Sequenz teilen.

12. Pharmazeutische Zusammensetzung gemäß Anspruch 11, wobei die Einzeldomänenantikörper, die gegen ein Target gerichtet sind, die gleiche Sequenz haben, oder wobei mindestens zwei Anti-Target-Einzeldomänenantikörper eine unterschiedliche Sequenz haben, aber gegen die gleichen Epitope oder unterschiedliche Epitope auf dem gleichen Target gerichtet sind.

13. Pharmazeutische Zusammensetzung gemäß einem der Ansprüche 1 bis 12, wobei
- die Anzahl der Anti-Serumprotein-Einzeldomänenantikörper mindestens zwei ist, sowie
- mindestens zwei Anti-Serumprotein-Einzeldomänenantikörper nicht die gleiche Sequenz teilen oder alle Serumprotein-Einzeldomänenantikörper die gleiche Sequenz teilen.

14. Pharmazeutische Zusammensetzung gemäß Anspruch 13, wobei die Einzeldomänenantikörper, die gegen ein Serumprotein gerichtet sind, die gleiche Sequenz haben, oder wobei mindestens zwei Anti-Serumprotein-Einzeldomänenantikörper eine unterschiedliche Sequenz haben, aber gegen die gleichen Epitope oder unterschiedliche Epitope des gleichen Serumproteins gerichtet sind.

15. Pharmazeutische Zusammensetzung gemäß einem der Ansprüche 1 bis 14, wobei das Serumprotein ausgewählt wird aus Serumalbumin, Serumimmunoglobulinen, Thyroxinbindendem Protein, Transferrin und Fibrinogen oder einem Fragment davon.

16. Pharmazeutische Zusammensetzung gemäß einem der Ansprüche 1 bis 15, wobei der Anti-Serumprotein-Einzeldomänenantikörper einer Sequenz entspricht, die durch eine beliebige der SEQ ID Nrn.: 1 bis 4 und 28 bis 40 wiedergegeben wird; oder
a) eine homologe Sequenz einer beliebigen der SEQ ID Nrn.: 1 bis 4 und 28 bis 40, umfassend. 1, 2, 3, 5, 6, 7, 8, 10 Additionen, Deletionen oder Substitutionen von einer oder mehreren Aminosäuren, die die funktionellen Eigenschaften des Einzeldomänenantikörpers nicht wesentlich ändern;
b) eine homologe Sequenz einer beliebigen der SEQ ID Nrn.: 1 bis 4 und 28 bis 40, mit einer Sequenzidentität von mehr als 70 %, 75 %, 80 %, 85 %, 90 %, 95 % oder 98 % mit SEQ ID Nrn.: 1 bis 4 und 28 bis 40;
c) ein funktioneller Abschnitt einer beliebigen der SEQ ID Nrn.: 1 bis 4 und 28 bis 40, der die Interaktion mit dem Target mit einer Affinität von 10⁻⁶ M oder besser aufrecht erhält;
d) ein funktioneller Abschnitt einer beliebigen der SEQ ID Nrn.: 1 bis 4 und 28 bis 40, der eine teilweise Deletion der vollständigen Aminosäuresequenz umfasst und dennoch die Bindestelle (n) und Proteindomäne(n) behält, die für Bindung und Interaktion mit dem Target notwendig sind.

17. Pharmazeutische Zusammensetzung gemäß einem der Ansprüche 1 bis 16, wobei das heterospezifische Polypeptidkonstrukt durch eine beliebige der SEQ ID Nrn.: 22 bis 24 wiedergegeben wird; oder
a) eine homologe Sequenz einer beliebigen der SEQ ID Nrn.: 22 bis 24, umfassend 1, 2, 3, 5, 6, 7, 8, 10 Additionen, Deletionen oder Substitutionen von einer oder mehreren Aminosäuren, die die funktionellen Eigenschaften des heterospezifischen Polypeptids nicht wesentlich ändern;
b) eine homologe Sequenz einer beliebigen der SEQ ID Nrn.: 22 bis 24, mit einer Sequenzidentität von mehr als 70 %, 75 %, 80 %, 85 %, 90 %, 95 % oder 98 % mit SEQ ID Nrn.: 22 bis 24;
c) ein funktioneller Abschnitt einer beliebigen der SEQ ID Nrn.: 22 bis 24, der die Interaktion mit dem Target mit einer Affinität von 10⁻⁶ M oder besser aufrecht erhält;
d) ein funktioneller Abschnitt einer beliebigen der SEQ ID Nrn.: 22 bis 24, der eine teilweise Deletion der vollständigen Aminosäuresequenz umfasst und dennoch die Bindestelle (n) und Proteindomäne(n) aufrecht erhält, die für Bindung und Interaktion mit dem Target notwendig sind.

18. Pharmazeutische Zusammensetzung gemäß einem der Ansprüche 1 bis 17, zum Behandeln, Vorbeugen und/oder • Lindern der Symptome einer Störung, die mit Allergien in Verbindung steht.

19. Pharmazeutische Zusammensetzung gemäß Anspruch 18, wobei die Störung ausgewählt wird aus Heuschnupfen, Asthma, atopischer Dermatitis, allergischer Hautreaktion, allergischen Augenreaktionen und Nahrungsmittelallergien.

## Revendications

1. Composition pharmaceutique pour une utilisation en thérapie comprenant :
- un ou plusieurs VHH de Camélidé ou VHH de Camélidé humanisés dirigés chacun contre une protéine sérique d'un sujet, et
- un ou plusieurs anticorps à domaine unique dirigés chacun contre une cible thérapeutique et/ou de diagnostic
et un véhicule pharmaceutiquement acceptable
**caractérisée en ce qu'**elle est administrée par inhalation.

2. Composition pharmaceutique selon la revendication 1, dans laquelle les anticorps à domaine unique dirigés contre une molécule cible sont des anticorps à domaine unique anti-IgE.

3. Composition pharmaceutique selon l'une quelconque des revendications 1 ou 2, qui est formulée sous la forme d'une pulvérisation nasale (par exemple aérosol), d'un inhalateur, d'un pulvérisateur de type à pompe ou d'un pulvérisateur d'aérosol.

4. Composition pharmaceutique selon l'une quelconque des revendications 1 à 3, qui est une formulation de VHH lyophilisée.

5. Composition pharmaceutique selon l'une quelconque des revendications 1 à 4, comprenant un support solide.

6. Composition pharmaceutique selon la revendication 5, dans laquelle le support solide est choisi parmi le talc, l'argile, la cellulose microcristalline, la silice et l'alumine.

7. Composition pharmaceutique selon l'une quelconque des revendications 1 à 4, comprenant un support liquide.

8. Composition pharmaceutique selon la revendication 7, dans laquelle le support liquide est choisi parmi l'eau, les hydroxyalkyles, les glycols et les mélanges d'eau-alcool/glycol.

9. Composition pharmaceutique selon l'une quelconque des revendications 1 à 8, dans laquelle les anticorps à domaine unique sont des anticorps à domaine unique dérivés d'un anticorps conventionnel à 4-chaînes (VH) ou dérivés d'un anticorps à chaîne lourde naturellement dépourvu de chaîne légère (VHH).

10. Composition pharmaceutique selon l'une quelconque des revendications 1 à 9, dans laquelle l'anticorps à domaine unique est humanisé.

11. Composition pharmaceutique selon l'une quelconque des revendications 1 à 10, dans laquelle
- le nombre d'anticorps à domaine unique anti-cible est d'au moins deux, et
- au moins deux anticorps à domaine unique anti-cible ne partagent pas la même séquence, ou tous les anticorps à domaine unique anti-cible partagent la même séquence.

12. Composition pharmaceutique selon la revendication 11, dans laquelle les anticorps à domaine unique qui sont dirigés contre une cible ont la même séquence ou dans laquelle au moins deux anticorps à domaine unique anti-cible ont une séquence différente mais sont dirigés contre les mêmes épitopes ou des épitopes différents sur la même cible.

13. Composition pharmaceutique selon l'une quelconque des revendications 1 à 12, dans laquelle
- le nombre d'anticorps à domaine unique anti-protéine sérique est d'au moins deux, et
- au moins deux anticorps à domaine unique anti-protéine sérique ne partagent pas la même séquence, ou tous les anticorps à domaine unique anti-protéine sérique partagent la même séquence.

14. Composition pharmaceutique selon la revendication 13, dans laquelle les anticorps à domaine unique qui sont dirigés contre une protéine sérique ont la même séquence ou dans laquelle au moins deux anticorps à domaine unique anti-protéine sérique ont une séquence différente mais sont dirigés contre les mêmes épitopes ou des épitopes différents sur la même protéine sérique.

15. Composition pharmaceutique selon l'une quelconque des revendications 1 à 14, dans laquelle la protéine sérique est choisie parmi l'albumine sérique, les immunoglobulines sériques, la protéine de transport de la thyroxine, la transferrine et, le fibrinogène ou un fragment de ceux-ci.

16. Composition pharmaceutique selon l'une quelconque des revendications 1 à 15, dans laquelle l'anticorps à domaine unique anti-protéine sérique correspond à une séquence représentée par l'une quelconque de SEQ ID NOs : 1 à 4 et 28 à 40 ; ou
a) une séquence homologue de l'une quelconque de SEQ ID NOs : 1 à 4 et 28 à 40, comprenant 1, 2, 3, 5, 6, 7, 8, 10 ajouts, délétions ou substitutions d'un ou plusieurs acides aminés, qui ne modifient pas sensiblement les caractéristiques fonctionnelles de l'anticorps à domaine unique ;
b) une séquence homologue de l'une quelconque de SEQ ID NOs . 1 à 4 et 28 à 40, avec une identité de séquence de plus de 70%, 75%, 80%, 85%, 90%, 95% ou 98% avec SEQ ID NO : 1 à 4 et 28 à 40 ;
c) une partie fonctionnelle de l'une quelconque de SEQ ID NOs . 1 à 4 et 28 à 40 qui maintient l'interaction avec la cible avec une affinité de 10⁻⁶ M ou mieux ;
d) une partie fonctionnelle de l'une quelconque de SEQ ID NO : 1 à 4 et 28 à 40, qui comprend une délétion partielle de la séquence d'acides aminés complète et conserve toujours le(s) site(s) de liaison et le(s) domaine(s) de protéine nécessaires pour la liaison de et l'interaction avec la cible.

17. Composition pharmaceutique selon l'une quelconque des revendications 1 à 16, dans laquelle la construction de polypeptide hétérospécifique est représentée par l'une quelconque de SEQ ID NOs : 22 à 24 ; ou
a) une séquence homologue de l'une quelconque de SEQ ID NOs : 22 à 24 comprenant 1, 2, 3, 5, 6, 7, 8, 10 ajouts, délétions ou substitutions d'un ou plusieurs acides aminés, qui ne modifient pas sensiblement les caractéristiques fonctionnelles du polypeptide hétérospécifique ;
b) une séquence homologue de l'une quelconque de SEQ ID NOs : 22 à 24 avec une identité de séquence de plus de 70%, 75%, 80%, 85%, 90%, 95% ou 98% avec SEQ ID NO : 22 à 24 ;
c) une partie fonctionnelle de l'une quelconque de SEQ ID NOs : 22 à 24 qui maintient l'interaction avec la cible avec une affinité de 10⁻⁶ M ou mieux ;
d) une partie fonctionnelle de l'une quelconque de SEQ ID NOs : 22 à 24 qui comprend une délétion partielle de la séquence d'acides aminés complète et conserve toujours le(s) site(s) de liaison et le(s) domaine(s) de protéine nécessaires à la liaison de et l'interaction avec la cible.

18. Composition pharmaceutique selon l'une quelconque des revendications 1 à 17, destinée à traiter, prévenir et/ou atténuer les symptômes d'un trouble ayant trait à des allergies.

19. Composition pharmaceutique selon la revendication 18, dans laquelle ledit trouble est choisi parmi le rhume des foins, l'asthme, la dermatite atopique, une réaction allergique cutanée, les réactions allergiques oculaires et les allergies alimentaires.
